# EUROPÄISCHE PATENTANMELDUNG

(11) **EP 4 512 382 A1**
(43) Veröffentlichungstag der Anmeldung: **26.02.2025**
(21) Anmeldenummer: 24196202.6
(22) Anmeldetag: 23.08.2024
(51) Int. Cl.: A61F 13/56, A61F 13/58

(54) **MECHANISCHES WINDELVERSCHLUSSBAND, HALBZEUGBAND UND WINDEL**

(30) Priorität: 25.08.2023 DE 102023122877
(71) Anmelder: Lohmann-koester, 96146 Altendorf (DE)
(72) Erfinder: Fryda, Matthias, 96146 Altendorf (DE); Schmidt, Dominik, 96146 Altendorf (DE); Keilholz, Clemens, 96146 Altendorf (DE)
(74) Vertreter: Reuther, Martin

(57) **Zusammenfassung**

Durch eine geeignete Abstimmung der Klebstoffschicht unter Berücksichtigung der Verhältnisse bei der Windelproduktion ist es möglich, dass die Klebstoffschicht eines Windelverschlussbands bei ausreichender Niederhaltefunktion des Windelverschlussbands auf eine textile Oberfläche einer Windel in einem Bereitstellungszustand und bei ausreichend stabiler Befestigung einer ersten Komponente eines zwei-komponentigen mechanischen Verschlusssystems dieses Windelverschlussbands nach dem Übergang von dem Bereitstellungszustand in einen Arbeitszustand eine ausreichende Verschlussfähigkeit des Windelverschlussbands an der Windel in dem Arbeitszustand nicht bzw. nur unwesentlich beeinträchtigt ist.

## Beschreibung

Die Erfindung betrifft ein mechanisches Windelverschlussband mit einer klebend wirksamen Niederhaltefunktion, wobei das Windelverschlussband ein sich entlang einer Länge erstreckendes Trägerband mit einem Fastening-Bereich und einem von dem Fastening-Bereich entlang der Länge des Trägerbands versetzt angeordneten Permanent-Bereich aufweist und wobei in dem Fastening-Bereich eine Komponente eines zwei-komponentigen mechanischen Verschlusssystems mittels einer Klebstoffschicht an dem Trägerband angebracht ist und diese Klebstoffschicht wenigstens einen offenen Bereich zu Ausübung der Niederhaltefunktion aufweist. Auch betrifft die Erfindung ein Halbzeugband zur Bereitstellung eines Windelverschlussbands, wobei das Windelverschlussband durch Trennen des Halbzeugbands parallel zur Länge des Windelverschlussbands aus dem Halbzeugband bereitstellbar ist und eine Windel mit einem derartigen Windelverschlussband. Ebenso betrifft die Erfindung eine Windel mit einem derartigen Windelverschlussband bzw. eine Windel mit wenigstens einem öffen- und wiederverschließbaren Windelverschlussband, welches ein sich entlang einer Länge erstreckendes Trägerband mit einem Fastening-Bereich und einem von dem Fastening-Bereich entlang der Länge des Trägerbands versetzt angeordneten Permanent-Bereich aufweist, wobei in dem Fastening-Bereich eine Komponente eines zwei-komponentigen mechanischen Verschlusssystems mittels einer Klebstoffschicht an dem Trägerband angebracht ist und diese Klebstoffschicht wenigstens einen offenen Bereich zu Ausübung einer klebend wirksamen Niederhaltefunktion des Windelverschlussbands aufweist, wobei das Windelverschlussband in einem Bereitstellungszustand einen Windelrand der Windel umgreifend sowohl in seinem Permanent-Bereich permanent an der Windel befestigt als auch in seinem Fastening-Bereich lösbar mit der Windel verbunden ist und wobei die Verbindung in dem Fastening-Bereich zu einem textilen Oberflächenbereich der Windel erfolgt und das Windelverschlussband durch Öffnen der Verbindung in dem Fastening-Bereich in einen Arbeitszustand verbringbar ist.

Windeln sind in sehr verschiedenen Bauformen am Markt zu finden. In einer besonders verbreiteten Bauform werden Windelverschlussbänder separat an an dem restlichen Windelkörper ausgeformten oder sogar an separaten Windelohren, die dann an dem Windelkörper befestigt sind, befestigt. Ebenso können derartige Windelverschlussbänder an gürtelartigen Einrichtungen der Windeln angebracht werden. Diese Windelverschlussbänder weisen einen Permanent-Bereich auf, mit welchem diese permanent an der Windel fixiert sind, was beispielsweise durch entsprechende Klebstoffe oder aber auch durch Verschweißungen oder ähnliche Verbindungsmaßnahmen realisiert sein kann. Darüber hinaus weisen derartige Windelverschlussbänder einen Fastening-Bereich auf, welcher eine verschließbare und öffenbare Verbindung mit geeigneten Bereichen, wie beispielsweise mit einer Landezone oder mit einer Windelaußenseite, der Windel eingehen kann, um auf diese Weise die Windel verschließen und dann auch wieder öffnen zu können. Je nach konkreter Situation kann ein derartiges Öffnen und Wiederverschließen erforderlich sein, wenn beispielsweise der Sitz der Windel nachgebessert oder aber das Innere der Windel geprüft werden soll. Die Windel wird hierbei in einem Zustand ausgeliefert, in welchem die Windelverschlussbänder möglichst einfach greifbar sein sollen, wozu diese in einem Bereitstellungszustand dem jeweiligen Nutzer bzw. Kunden präsentiert werden. Der Kunde kann die Windelverschlussbänder dann ergreifen und in einem Arbeitszustand verbringen, in welchem dann die Windel entsprechend wieder öffenbar verschlossen werden kann. Je nach konkreter Umsetzung können durch die Windelverschlussbänder nicht nur ein Verschließen der Windel sondern auch eine Fixierung der Windel in einer Entsorgungsposition realisiert werden.

Windelverschlussbänder bzw. Halbzeugbänder mit einer klebend wirksamen Niederhaltefunktion und einem zwei-komponentigen mechanischen Verschlusssystem, mit denen insbesondere gegebenenfalls auch shear- und peel-Funktionen gezielt getrennt umgesetzt werden, offenbaren beispielsweise die WO 2005 / 074 852 A1 und die EP 3 062 755 B1. Beide Druckschriften und auch die EP 3 409 253 B1 offenbaren, dass derartige Windelverschlussbänder um die Kante bzw. den Windelrand einer Windel oder eines Windelohrs in einem Bereitstellungszustand herumgefaltet werden können. Hierbei ist es, wie beispielsweise die BR 20 2018 073 992 U2 und die EP 3 125 843 B1 erläutern, von Bedeutung, die Windelverschlussbänder in dem Bereitstellungszustand, in welchem diese beispielsweise um ein Windelohr oder den Windelrand herumgefaltet werden, insbesondere während der Produktion und des Verpackens der Windeln betriebssicher zu halten, was letztlich auch unmittelbar nachvollziehbar ist, da Windeln als Massenprodukte nur maschinell und mit hohen Produktionsgeschwindigkeiten wirtschaftlich bereitzustellen sind. Wenn dann derartige Windelverschlussbänder während der Produktion bzw. vor dem Verpacken aufspringen bzw. ihren Bereitstellungszustand verlassen, führt dieses in der Regel zumindest zu einem unansehnlichen Äußeren der Windel, wenn diese benutzt werden soll. Insbesondere kann ein Verlassen des Bereitstellungszustands zur Unzeit auch die Handhabe der Windel durch den Nutzer unbillig erschweren oder sogar den Produktions- bzw. Verpackungsprozess stören.

Wie beispielsweise die DE 10 2013 101 886 A1 darlegt, kann ein Wiederverschließen der Windelverschlussbänder, was beispielsweise nach einem Kontrollblick, ob eine Windel gefüllt ist oder nicht, oder bei einem schlechten Sitz der Windel durchgeführt werden muss, behindert werden, wenn klebende oder mechanische Verschlusselemente durch herausgerissene Materialreste blockiert werden. Während die BR 20 2018 073 992 U2 und die DE 10 2013 101 886 A1 ein Ausfransen bzw. Kontaminieren des Fastening-Bereichs durch herausgerissene Fasern aus der Landezone an der Windelaußenseite, in welcher das Windelverschlussband mit seinem Fastening-Bereich lösbar befestigt wird, zu vermeiden suchen, beschäftigt sich die EP 3 125 843 B1 explizit mit der Problematik, dass Fasern aus der Innenseite eines Windelohrs delaminiert werden, wenn das Windelverschlussband von seinem Bereitstellungszustand in den Arbeitszustand überführt wird. Ähnlich wie bei der in der BR 20 2018 073 992 U2 offenbarten Lösung wird auch bei der in der EP 3 125 843 B1 offenbarten Lösung auf einen ergänzenden Klebestreifen gesetzt, welcher die klebende Niederhaltefunktion in dem Bereitstellungszustand ermöglichen soll. Hierbei soll entsprechend der Offenbarung der EP 3 125 843 B1 der ergänzende Klebestreifen auch in dem Arbeitszustand klebend wirken, während etwa frei liegende Bereiche einer Klebstoffschicht, mit der das Hakenmaterial des mechanischen Verschlusssystems an dem Windelverschlussband befestigt ist, nur während des Produktionsprozesses klebend wirksam sein sollen. Umgekehrt soll nach der Lehre der BR 20 2018 073 992 U2 der ergänzende Klebestreifen lediglich während des Produktionsprozesses wirksam sein, während eine gegebenenfalls frei liegende Bereiche aufweisende Klebstoffschicht, mit der im Übrigen das Hakenmaterial des mechanischen Verschlusssystems an dem Windelverschlussband befestigt ist, zu einem Delaminieren oder Ausreißen von Fasern führen können. Auch die EP 3 409 253 B1 schlägt vor, die Klebstoffschicht, mit der im Übrigen das Hakenmaterial des mechanischen Verschlusssystems an dem Windelverschlussband befestigt ist, insbesondere wenn diese frei liegende Bereiche aufweist, derart auszugestalten, dass die frei liegenden Bereiche nach dem Produktionsprozess möglichst keine klebende Wirkung mehr entfalten.

Einen anderen Ansatz verfolgt die EP 0974 326 B1, nach welcher das Windelverschlussband an dem Windelohr auf sich selbst umgefaltet in dem Bereitstellungszustand vorgehalten werden soll, wobei in einem Arbeitszustand des Verschlussbandes ein offener Bereich einer Klebstoffschicht auf einem speziellen Realease-Band oder auf einem Bereich des Trägerbandes zwischen dem mechanischen Element und dem Release-Band bereitgestellt wird. Dieser offene Bereich kann dann auch bei einem Verschließen bzw. Wiederverschließen der Windel in einem Arbeitszustand ergänzend zur Verschlussfähigkeit beitragen, wobei eine Antihaftbeschichtung der Fasern einer entsprechenden Landezone auf der Windelaußenseite für eine Wiederverschließbarkeit als essentiell angesehen wird.

Es ist Aufgabe vorliegender Erfindung, derartige mechanische Windelverschlussbänder sowie entsprechende Halbzeugbänder und Windeln bereitzustellen, bei welchen bei ausreichender Niederhaltefunktion in dem Bereitstellungszustand und bei ausreichend stabiler Befestigung der Komponente des zwei-komponentigen mechanischen Verschlusssystems nach dem Übergang von dem Bereitstellungszustand in den Arbeitszustand eine ausreichende Verschlussfähigkeit gewährleistet werden kann.

Die Aufgabe der Erfindung wird durch Windelverschlussbänder, Halbzeugbänder und Windeln mit den Merkmalen der unabhängigen Ansprüche gelöst. Weitere, gegebenenfalls auch unabhängig hiervon, vorteilhafte Ausgestaltungen finden sich in den Unteransprüchen sowie der nachfolgenden Beschreibung.

Hierbei geht die Erfindung von der Grunderkenntnis aus, dass es durch eine geeignete Abstimmung der Klebstoffschicht unter Berücksichtigung der Verhältnisse bei der Windelproduktion möglich ist, dass die Klebstoffschicht eines Windelverschlussbands bei ausreichender Niederhaltefunktion und bei ausreichend stabiler Befestigung der Komponente des zwei-komponentigen mechanischen Verschlusssystems nach dem Übergang von dem Bereitstellungszustand in den Arbeitszustand eine ausreichende Verschlussfähigkeit in dem Arbeitszustand nicht bzw. nur unwesentlich beeinträchtigt.

So kann sich ein mechanisches Windelverschlussband mit klebend wirksamer Niederhaltefunktion, welches ein sich entlang einer Länge erstreckendes Trägerband mit einem Fastening-Bereich und einem von dem Fastening-Bereich entlang der Länge des Trägerbands versetzt angeordneten Permanent-Bereich aufweist, wobei in dem Fastening-Bereich eine erste Komponente eines zwei-komponentigen mechanischen Verschlusssystems mittels einer Klebstoffschicht an dem Trägerband angebracht ist und diese Klebstoffschicht wenigstens einen offenen Bereich zur Ausübung der Niederhaltefunktion aufweist, dadurch auszeichnen, dass die Klebstoffschicht ein Speichermodul *G'* von mehr als 3,3 10⁵ Pa bei 25 °C und eine Glasübergangstemperatur von wenigstens 23°C aufweist, um bei ausreichender Niederhaltefunktion in dem Bereitstellungszustand und bei ausreichend stabiler Befestigung der Komponente zwei-komponentigen mechanischen Verschlusssystems nach dem Übergang von dem Bereitstellungszustand in den Arbeitszustand eine ausreichende Verschlussfähigkeit gewährleisten zu können. Durch den Einsatz eines derartig ausgebildeten Klebstoffes als Klebstoffschicht, mittels welcher die erste Komponente des zwei-komponentigen mechanischen Verschlusssystems an dem Trägerband angebracht ist, kann gewährleistet werden, dass diese erste Komponente ausreichend stabil an dem Trägerband befestigt ist, wobei sich herausgestellt hat, dass dieses nicht nur während des Herstellungsprozesses gilt, bei welchem naturgemäß höhere Temperaturen, als Raumtemperatur herrschen, sondern auch nach einer Lagerung und bei der Benutzung einer entsprechend ausgestatteten Windel, wenn das Windelverschlussband von einem Bereitstellungszustand in einen Arbeitszustand verbracht und für ein wiederöffenbares Verschließen der Windel genutzt wird. Das Speichermodul *G'* liegt oberhalb des Dahlquist-Kriteriums. Das Dahlquist-Kriterium findet sich unter anderem auch diskutiert in dem Artikel von D. Budelmann, C. Schmidt und D. Meiners "adhesion-cohesion balance of prepreg tack in thermoset automated fiber placement (Part 1: Adhesion and surface wetting; Composite part C: Open access 6 (2021) 100204; veröffentlicht von Elsevier B. V.). Ebenso wird das Dahlquist-Kriterium von D. Satas im Handbook of pressure sensitive adhesived technology, 2nd ed. (1989), S. 172 bis 176 und in der WO 2016 / 209 651 A1 bzw. in der US 3 787 531 diskutiert. Hierbei erfolgen die entsprechenden Messungen des Speichermoduls *G'* zur Einordnung bezüglich des Dahlquist-Kriteriums bei Raumtemperatur und einer Frequenz von 1 Hz, wobei die Raumtemperatur in dem vorliegenden Zusammenhang bei 25°C definiert ist. Entsprechend dieses Dahlquist-Kriteriums gilt der vorliegend vorgeschlagene Klebstoff also als nicht klassischer Haftklebstoff. Trotzdem erzielt der vorgeschlagene Klebstoff eine ausreichende Niederhaltefähigkeit, welche insbesondere eine ausreichend stabile Befestigung der ersten Komponente des zwei-komponentigen mechanischen Verschlusssystems gewährleistet und dennoch die Zahl der Faserausrisse derart niedrig lässt, dass eine Kontamination durch Fasern sowohl der offenen Klebefläche als auch der ersten Komponente des zwei-komponentigen mechanischen Verschlusssystems auf ein ausreichendes Minimum begrenzt werden kann. Auch die hohe Glasübergangstemperatur scheint zunächst dafür zu sprechen, dass der vorgeschlagene Klebstoff als nicht klebrig anzusehen sein müsste. Anscheinend scheinen die Verhältnisse an einer Windelmaschine jedoch beispielsweise hinsichtlich ihres Temperaturbereichs ausreichend erhöht, um dem Klebstoff dennoch ausreichend klebrige Eigenschaften zu verleihen.

Alternativ bzw. kumulativ hierzu kann sich ein mechanisches Windelverschlussband mit klebend wirksamer Niederhaltefunktion, welches ein sich entlang einer Länge erstreckendes Trägerband mit einem Fastening-Bereich und einem von dem Fastening-Bereich entlang der Länge des Trägerbands versetzt angeordneten Permanent-Bereich aufweist, wobei in den Fastening-Bereich eine erste Komponente eines zwei-komponentigen mechanischen Verschlusssystems mittels einer Klebstoffschicht an dem Trägerband angebracht ist und diese Klebstoffschicht wenigstens einen offenen Bereich zur Ausübung der Niederhaltefunktion aufweist, dadurch auszeichnen, dass die Klebstoffschicht eine Sofortklebekraft zwischen 1,0 N/(25 mm) und 5,0 N/(25 mm) und eine Klebkraft zwischen 10,0 N/(25 mm) und 25,0 N/(25 mm) aufweist. Es hat sich herausgestellt, dass ein derartig ausgestalteter Klebstoff, der zu einer Klebstoffschicht ausgeformt ist, um eine erste Komponente eines zwei-komponentigen mechanischen Verschlusssystems an dem Trägerband anzubringen bzw. zu befestigen, zum einem eine ausreichende Haltekraft aufweist, um die erste Komponente in ausreichendem Maße an dem Trägerband zu befestigen, dass das auf diese Weise gebildete Windelverschlussband seine Verschlussfunktion, selbst wenn die Windel mehrfach geöffnet und wieder verschlossen werden soll, erfüllen kann. Darüber hinaus ermöglichen die offenen Bereiche dieser Klebstoffschicht immer noch eine ausreichende Niederhaltefunktion in dem Bereitstellungszustand, sodass bei ausreichend stabiler Befestigung der Komponente des zwei-komponentigen mechanischen Verschlusssystems nach dem Übergang von Bereitstellungszustand in den Arbeitszustand eine ausreichende Verschlussfähigkeit gewährleistet werden kann, wobei dennoch eine etwaige Kontamination des Fastening-Bereichs durch ausgerissene Fasern minimiert werden kann.

Hierbei hat sich herausgestellt, dass durch ein Speichermodul *G'* und eine geeignete Glasübergangstemperatur einerseits bzw. eine entsprechende Abstimmung der Sofortklebekraft und der Klebkraft andererseits bei einer auch im Übrigen geeigneten Ausgestaltung des Windelverschlussbands es ermöglichen, dass die offenen Bereiche der Klebestoffschicht, mit welcher die erste Komponente an dem Trägerband angebracht ist, zwar ihre Niederhaltefunktion insbesondere während der Produktion, in der Regel aber auch noch bis zu dem Zeitpunkt, zu welchem ein Nutzer dann das Windelverschlussband von seinem Bereitstellungszustand in den Arbeitszustand verbringen muss, ausreichend erfüllen kann, ohne dass beim erstmaligen Öffnen durch die offenen Bereiche zu viele Faserausrisse aus einem textilen Oberflächenbereich der Windel, an welchem der Fastening-Bereich in dem Bereitstellungszustand lösbar mit der Windel verbunden ist, stattfinden. Dieses bedingt, dass etwaige ausgerissene Fasern die Funktionalität des Windelverschlussbandes in seinem Arbeitszustand nicht bzw. nur unwesentlich beeinträchtigen, was beispielsweise dadurch bedingt sein kann, dass durch derartige Faserausrisse nicht nur die offenen Bereiche, sondern auch die erste Komponente verdeckt sein können, welche dann die Funktionalität beinträchtigen.

Dementsprechend kann sich eine Windel mit wenigstens einem öffen- und wiederverschließbaren Windelverschlussband, welches ein sich entlang einer Länge erstreckendes Trägerband mit einem Fastening-Bereich und einem von den Fastening-Bereich entlang der Länge des Trägerbands versetzt angeordneten Permanent-Bereich aufweist, wobei in dem Fastening-Bereich eine erste Komponente eines zwei-komponentigen mechanischen Verschlusssystems mittels einer Klebstoffschicht an dem Trägerband angebracht ist und diese Klebstoffschicht wenigsten einen offenen Bereich zur Ausübung einer klebend wirksamen Niederhaltefunktion des Windelverschlussbands aufweist, wobei das Windelverschlussband in einem Bereitstellungszustand einen Windelrand der Windel umgreifend sowohl in seinem Permanent-Bereich permanent an der Windel befestigt als auch in seinem Fastening-Bereich lösbar mit der Windel verbunden ist und wobei die Verbindung in dem Fastening-Bereich zu einem textilen Oberflächenbereich der Windel erfolgt und das Windelverschlussband durch Öffnen der Verbindung in dem Fastening-Bereich in einen Arbeitszustand verbringbar ist, dadurch auszeichnen, dass die Klebstoffschicht einem Öffnen der Verbindung eine peel-Mindestkraft von wenigstens 0,6 N/(25 mm) entgegensetzt und bei dem Öffnen weniger als 20 Fasern pro 0,125 cm² aus dem textilen Oberflächenbereich ausreist, um bei ausreichender Niederhaltefunktion in dem Bereitstellungszustand und bei ausreichend stabiler Befestigung der Komponente des zwei-komponentigen mechanischen Verschlusssystems nach dem Übergang von dem Bereitstellungszustand in den Arbeitszustand eine ausreichende Verschlussfähigkeit gewährleisten zu können. Die sehr geringe Menge ausgerissener Fasern ermöglicht es bei einer ausreichenden Niederhaltefunktion in dem Bereitstellungszustand eine ausreichende Verschlussfähigkeit des Windelverschlussbands in den Arbeitszustand zu gewährleisten, in dem eine Kontamination des Fastening-Bereichs aufgrund durch die offenen Bereiche der Klebstoffschicht ausgerissener Fasern auf ein ausreichendes Minimum reduziert werden kann. Erstmals kann mithin durch die Reduktion der Zahl der ausgerissenen Fasern eine ausreichende Verschlussfähigkeit nach einem Verbringen des Windelverschlussbandes in den Arbeitszustand gewährleistet werden.

Windeln sind am Markt an sich als Massenprodukt hinlänglich bekannt und dienen der Körperhygiene, insbesondere bei Säuglingen und Kleinkindern, aber mehr und mehr auch bei Erwachsenen. Hierbei weisen moderne Windeln in der Regel einen zentralen Windelkörper auf, welcher eine Außenschicht und eine Innenschicht sowie einen von diesen Schichten eingeschlossenen saugfähigen Kern umfasst. Hierbei ist in der Regel die Außenschicht feuchtigkeitsundurchlässig ausgestaltet, während die Innenschicht dazu dient, etwaige Flüssigkeiten von dem Körper weg zu dem Windelkern zu führen und in der Regel feuchtigkeitsdurchlässig ausgebildet ist. Schon wegen des unmittelbaren Hautkontakts weist die Innenschicht in der Regel einen textilen Charakter auf, welcher sich in der Praxis als hautfreundlich bewährt. Häufig finden sich auch an der Außenschicht textile Strukturen, welche - je nach konkreter Ausgestaltung der Windel - die gesamte Außenschicht, oder zumindest sehr große Teile hiervon überdecken oder lediglich in begrenzten Bereichen, beispielsweise als sogenanntes Frontal-Tape lediglich als zweite Komponente des zwei-komponentigen mechanischen Verschlusssystems dienen, mittels dessen die Windel am jeweiligen Körper verschlossen und wieder geöffnet werden kann.

Die Außenschicht und die Innenschicht treffen sich bei derartigen Windeln den Windelkern umgehend außerhalb desselben, so dass Flüssigkeiten sicher in dem Windelkern eingeschlossen werden können.

Dem Schließen und Öffnen der Windel dienen häufig Windelverschlussbänder, welche an geeigneten Positionen an den Windelaußenrändern bzw. in der Nähe von Windelkanten, oder aber unmittelbar an Windelohren, die - je nach konkreter Umsetzung - ihrerseits separat an den Windelkörpern angesetzt oder aus der Außenschicht bzw. aus der Innenschicht ausgeformt sind, angesetzt sind. Hierbei erfolgt das Ansetzen der Windelverschlussbänder in der Regel durch eine permanente Verbindung derselben in deren Permanent-Bereich, während derartige Windelverschlussbänder noch einen Fastening-Bereich aufweisen, mittels dessen ein lösbares Verschließen der Windeln möglich ist.

Dadurch, dass der Fastening-Bereich und der Permanent-Bereich entlang der Länge des Trägerbands versetzt angeordnet sind, kann das Windelverschlussband mit diesem Trägerband von dem Permanent-Bereich ausgehend und übergreifend an geeigneten Landezonen an der Windel ansetzen, um auf diese Weise in dem Arbeitszustand die Windel öffenbar verschließen zu können. Die Länge und die Breite des Windelverschlussbands und des Trägerbands definieren sich vorliegend insbesondere in der Ebene des Trägerbands bzw. in einer hierzu parallelen Ebene. Dadurch, dass der Permanent-Bereich und der Fastening-Bereich entlang der Länge des Trägerbands versetzt angeordnet sein sollen, ergibt dieser Versatz, wenn in der Ebene des Trägerbands senkrecht zur Länge auf das Windelverschlussband gesehen wird, dass sich die beiden Bereiche nicht überlappen. Wird das Windelverschlussband aus einem Halbzeugband bereitgestellt, so kann, je nach konkreter Ausgestaltung des Halbzeugbands die Länge des Trägerbands senkrecht zu einer Maschinenlaufrichtung bzw. parallel zu einer Querrichtung des Halbzeugbands und die Breite des Trägerbands parallel zur Maschinenlaufrichtung bzw. senkrecht zur Querrichtung des Halbzeugbands definiert sein. Auch der Versatz der beiden Bereich erfolgt dann in Querrichtung bzw. senkrecht zur Maschinenlaufrichtung des Halbzeugbands.

Als Trägerband kann vorliegend jede bandartige Struktur dienen, welche eine Länge aufweist und senkrecht zu dieser Länge eine Breite umfasst, wobei die Länge und die Breite vorzugsweise wesentlich größer sind als die Dicke, sodass dem Trägerband eine bandartige Struktur verliehen werden kann. Je nach konkreter Umsetzung kann die Breite größer als die Länge des Windelverschlussbands sein, insbesondere wenn das Windelverschlussband auch Funktionen eines Windelohrs übernehmen soll. Häufig werden Windelverschlussbänder jedoch - schon aus Kostengründen - verhältnismäßig schmal, also mit einer Breite, die kleiner ist als die Länge, ausgebildet. Für den vorliegenden Zusammenhang spielt die konkrete Breite des Windelverschlussbandes zunächst keine Rolle, solange das Windelverschlussband ausreichend lang ist, um den Fastening-Bereich und den Permanent-Bereich versetzt zueinander bereitstellen zu können.

Vorliegend bezeichnet der Permanent-Bereich den Bereich des Trägerbands bzw. des Windelverschlussbands, der dazu ausgebildet und bestimmt ist, permanent an der Windel befestigt zu werden. Dementsprechend kann dort beispielsweise eine Klebstoffschicht vorgesehen sein, welche in ihrer Klebkraft darauf abgestimmt ist, dass das Windelverschlussband bzw. das Trägerband während des normalen Gebrauchs der Windel permanent und mit einer ausreichenden Betriebssicherheit in seiner Position zu bleiben, wobei es denkbar ist, dass durch aufwendige Lösemaßnahmen durchaus eine Trennung dennoch möglich sein könnte, wobei dieses nichts an der Funktionalität und an der Permanenz der Befestigung ändert, solange während eines normalen Gebrauchs der Windel eine permanente Befestigung gewährleistet ist. Alternativ kann für eine derartige permanente Befestigung jede geeignete Verbindungsart, wie beispielsweise eine durch Ultraschall bereitgestellte Befestigung, eine Befestigung durch Laserschweißen oder Ähnliches, in dem Permanent-Bereich zur Anwendung kommen.

Der zu dem Permanent-Bereich entlang der Länge des Trägerbands versetzt angeordnete Fastening-Bereich definiert einen Bereich des Trägerbandes bzw. des Windelverschlussbands, welcher eine lösbare Verbindung mit der Windel eingehen kann. Die Lösbarkeit der Verbindung ermöglicht es, dass ein Nutzer das Windelverschlussband bzw. das Trägerband ergreifen und von der Windel lösen kann, um dieses beispielsweise von dem Bereitstellungszustand in den Arbeitszustand verbringen zu können. Ebenso ermöglicht diese Lösbarkeit, dass die Windel durch Ergreifen des Fastening-Bereiches und dessen Lösen von der Windel geöffnet und wiederverschlossen werden kann, was beispielsweise für eine Kontrolle des Sitzes der Windel oder deren Befüllung oder auch für eine Entsorgung der Windel entsprechend benutzt werden kann.

Je nach konkreter Umsetzung kann in dem Fastening-Bereich beispielsweise eine geeignet ausgebildete Klebeschicht vorgesehen sein, welche darauf abgestimmt ist, eine lösbare Verbindung zu bestimmten Bereichen der Windel, beispielsweise zu der Außenschicht oder zu sonstigen bestimmten Landezonen einzugehen, so dass auf diese Weise die entsprechend der Funktionalität umgesetzt sein kann. Vorliegend jedoch soll in dem Fastening-Bereich zumindest eine erste Komponente eines zwei-komponentigen mechanischen Verschlusssystems angeordnet sein, sodass der Fastening-Bereich zumindest teilweise eine mechanische Verbindung mit hierfür geeigneten Bereichen der Windel eingehen können soll, welche dann dementsprechend auch mechanisch lösbar ausgebildet sein soll. Dementsprechend tragen dann derartige Bereiche der Windel vorzugsweise die zweite Komponente des zwei-komponentigen mechanischen Verschlusssystems.

Zwei-komponentige mechanische Verschlusssysteme sind in ihrer Gesamtheit vielfältig am Markt bekannt, beispielsweise als Haken-Schlaufen-Verschlüsse, als Haken-Ösen-Verschlüsse, als Knöpfe und Knopflöcher oder als ineinander hakende Haken-Haken-Anordnungen, so dass jeweils eine dieser Baugruppen als erste Komponente und die andere dieser Baugruppen als zweite Komponente der jeweils zwei-komponentigen mechanischen Verschlusssysteme angesehen werden können. Entsprechende Anordnungen sind insbesondere als Haken-Schlaufen-Verschlüsse unter verschiedenen Markennamen auch für Windeln bzw. Windelverschlussbänder hinlänglich am Markt bekannt, wobei im Konkreten durchaus Haken-in-Haken bzw. Haken-in-Schlaufe oder Haken-in-textile-Anordnung entsprechende mechanische Verschlusssysteme darstellen, welche für Windel bzw. Windelverschlussbändern zu Anwendung kommen. Rein faktisch spielt es jeweils zunächst keinerlei Rolle, welche der beiden Komponenten als erste und welche der beiden Komponenten als zweite Komponente des zwei-komponentigen mechanischen Verschlusssystems bezeichnet wird.

Häufig stellen bei derartigen mechanischen Verschlusssystemen etwaige Haken einen kostenträchtigen Anteil dar, so dass Tendenzen zu sehen sind, die Haken möglichst einfach auszugestalten. Hierbei hat es sich am Markt bewährt, neben dem mechanischen Verschlusssystem noch klebende Momente dem Verschluss beizugeben, wie beispielsweise eine ergänzende klebend wirksame Niederhaltefunktion. Hierbei wird häufig davon ausgegangen, dass die klebend wirksame Niederhaltefunktion in Wesentlichen peel-off-Kräften widerstehen soll, während die möglichst einfach ausgebildeten Haken im Wesentlichen shear-Kräften widerstehen sollen. Shear-Kräfte finden sich dann in der Regel parallel zu den Trägerbändern bzw. zu der wesentlichen Erstreckung der Windelverschlussbänder, während peel-off-Kräfte im Wesentlichen in der Regel parallel zur Dicke bzw. senkrecht zur Breite und Länge der Trägerbänder bzw. der Windelverschlussbänder auftreten. Eine derartige Ausgestaltung ermöglicht es, die entsprechende Komponente des zwei-komponentigen mechanischen Verschlusssystems, also beispielsweise die Haken, verhältnismäßig einfach und kostengünstig auszubilden, beispielsweise sogar lediglich als senkrecht hervorstehende Nadeln bzw. Spitzen, welche in die zweite Komponente des zwei-komponentigen mechanischen Verschlusssystems eindringen, so dass diese shear-Kräften, also Kräften senkrecht zu der Ausrichtung dieser Spitzen bzw. Nadeln, widerstehen können, während durch die klebend wirksamen Niederhaltefunktion dann im Wesentlichen den peel-off-Kräften, also den parallel zu den Nadeln bzw. Spitzen auftretenden Kräfte widerstanden werden kann. Hierbei versteht es sich, dass die entsprechenden Vorteile auch dann zu finden sind, wenn die erste Komponente des zwei-komponentigen mechanischen Verschlusssystems etwas komplexer, also beispielsweise mit kleinen Hakenausformungen oder Widerhaken o.ä., ausgebildet ist.

Insbesondere in der Kategorie zwei-komponentiger mechanischer Verschlusssysteme mit Haken als eine der Komponenten haben sich textile Oberflächenbereiche als zweite Komponente als vorteilhaft erwiesen, da derartige textile Oberflächenbereiche verhältnismäßig kostengünstig bereitstellbar sein können, eine angenehme haptische Oberfläche bereitstellen und in einer Vielzahl unterschiedlicher Ausgestaltungen zur Verfügung stehen, um auf diese Weise individuellen Erfordernissen an die Erscheinung der Windeln gerecht werden zu können.

Wie bereits vorstehend angedeutet, können entsprechende textile Oberflächenbereiche, solange sie als Komponente des zwei-komponentigen mechanischen Verschlusssystems genutzt werden sollen, gegebenenfalls verhältnismäßig großflächig an der Windel oder aber lediglich in definierten Landezonen, wie beispielsweise an einem Frontaltape, vorgesehen sein. Hierbei liegt es in der Natur der Sache, dass kleinere Landezonen gegebenenfalls etwas aufwendiger und teurer hinsichtlich ihrer konstruktiven Ausgestaltung gewählt werden können, während eine großflächige Ausgestaltung engere Grenzen unter Berücksichtigung der Kosten setzt. Unabhängig hiervon können jedoch im vorliegenden Zusammenhang textile Oberflächenbereiche aus den gesamten Gebiet der Textilien bis hin zu textilartigen Kunststoffen bzw. Zellulose umfassenden Strukturen gewählt werden, solange diese den Anforderungen an eine Windel allgemein hinsichtlich ihrer Materialeigenschaften, hinsichtlich ihrer Entsorgungseigenschaften bzw. hinsichtlich ihrer Hautverträglichkeit in ausreichendem Maße gerecht werden und im Übrigen in ausreichenden Maße in der Lage sind mit der anderen Komponente des zwei-komponentigen mechanischen Verschlusssystems einen ausreichend stabilen aber lösbaren Verschluss zu bilden.

Insbesondere können textile Oberflächenbereiche durch Gewebe oder Gewirke, insbesondere auch durch Nonwoven, entsprechend breitgestellt werden, wobei derartige Textilien beispielsweise aus Stapelfasern, kardierten Fasern, glatten oder gekräuselten Fasern, Garnen, Einzelfasern, Endlosfasern oder Fasern, die in geeigneter Weise zu einer flächigen Struktur verdichtet und/oder verbunden sind, gebildet werden, solange diese Struktur eine Wechselwirkung mit der anderen Komponente des zwei-komponentigen mechanischen Verschlusssystems zulässt. Hierbei versteht es sich, dass die letzte Bedingung lediglich für die Bereiche zutreffen muss, in welchen tatsächlich bestimmungsgemäß eine entsprechende verschließende Wirkung stattfinden können soll. Im Übrigen können entsprechende textile Bereiche auch von dieser Bedingung abweichend ausgebildet sein, was andererseits insbesondere nicht ausschließt, dass große Teile der textilen Bereiche, wenn nicht sogar alle textilen Bereiche, einer Windel dieser Bedingung genügen können.

Wie bereits eingangs angedeutet, werden Windeln und auch Windelverschlussbänder maschinell als Massenware bereitgestellt, so dass insbesondere die Verarbeitungsgeschwindigkeit und der Produktionsablauf, aber auch die zugehörigen Materialkosten von erheblicher Bedeutung sind. Je nach konkretem Herstellungsprozess werden hierbei die mechanischen Windelverschlussbänder während der Herstellung der Windeln an der Windel mit ihrem Permanent-Bereich appliziert und um einen Windelrand der Windel herum in einen Bereitstellungszustand derart an der Windel befestigt, dass auch der Fastening-Bereich eine Verbindung mit der Windel eingeht. Hierbei spielt es an sich keine Rolle, ob der Fastening-Bereich und der Permanent-Bereich gleichzeitig entsprechend an der Windel appliziert werden, ob dieses sukzessiv erfolgt.

Häufig wird hierbei das jeweilige Windelverschlussband, bevor es an der Windel appliziert wird, von einem Halbzeugband abgetrennt, wobei es sich in der Praxis herausgestellt hat, dass derartige Halbzeugbänder vorzugsweise entlang einer Maschinenlaufrichtung durchlaufend produziert werden, indem beispielsweise Klebstoffschichten im Permanent-Bereich und im Fastening-Bereich sowie entsprechende Komponenten des zwei-komponentigen mechanischen Verschlusssystems und sonstigen Komponenten, wie beispielsweise ein Fingerlift, mit dem bzw. auf das Trägerband aufgebracht werden.

Je nach konkreter Umsetzung kann die Herstellung des Halbzeugbands mehrnutzig erfolgen, so dass es nicht ausreicht, dass Windelverschlussband durch Trennen des Halbzeugbands parallel zur Länge des Windelverschlussbands bzw. senkrecht zur Maschinenlaufrichtung bzw. parallel zu einer senkrecht zu Maschinenlaufrichtung ausgerichteten Querrichtung zu durchtrennen, sondern auch die einzelnen Nutzen parallel zu Maschinenlaufrichtung in einzelne Windelverschlussbänder abgetrennt werden müssen. Hierbei ist die Reihenfolge dieser Trennungsschritte vorzugsweise an den Produktionsprozess abgepasst, wobei es in der Praxis sich insbesondere als erfolgreich herausgestellt hat, auch das Trennen der einzelnen Nutzen an der Windelmaschine durchzuführen.

Die entsprechenden Trennungsschritte können - je nach konkreter Umsetzung des Fertigungsprozesses - vor dem Aufwickeln des Halbzeugbands nach dessen Fertigung erfolgen, wenn lediglich die einzelnen Nutzen getrennt werden sollen. Ebenso kann die entsprechende Trennung erst an der Windelmaschine erfolgen, kurz bevor aus dem Halbzeugband letztlich die Windelverschlussbänder bereitgestellt und an der Windel appliziert werden.

Es versteht sich, dass gegebenenfalls an der Windelmaschine noch ergänzende Applikationen an dem Windelverschlussband vorgenommen werden können, wenn dieses für den Fertigungsvorgang vorteilhaft erscheint. Dieses können insbesondere beispielsweise besondere Applikationen, wie beispielsweise individualisierende Fingerlifts oder Ähnliches, sein.

Das Aufwickeln des Halbzeugbands kann hierbei - je nach konkreten Erfordernissen - in Form einer Rolle oder einer Spule erfolgen, wobei bei einer Rolle die einzelnen Lagen der Wickel senkrecht übereinander liegen, während bei einer Spule das Halbzeugband in einem Winkel senkrecht zur Achse der entsprechenden Wicklung aufgespult wird, wobei in der Regel der Winkel periodisch in seiner Richtung verändert wird, um auf dieser Weise einen kompakten Spulenkörper bereitstellen zu können. Derartige Wicklungen sind allerdings nicht zwingend notwendig. Es ist auch denkbar, dass die entsprechenden Halbzeugbänder lediglich lose und frei, beispielsweise in geeigneten Kisten, vorgehalten und zwischengelagert werden.

Der Produktionsprozess der Windeln erfolgt bei sehr hohen Maschinengeschwindigkeiten, so dass auch hohe Anforderungen an die Prozesssicherheit gestellt werden. Dementsprechend ist es erforderlich, dass ein mechanisches Windelverschlussband, wenn es einmal an der Windel appliziert ist, betriebssicher in der Position verbleibt, welche für den Produktionsprozess vorgesehen ist. Hierbei wird die Windel, nachdem sie produziert ist, insbesondere noch verkaufsfertig gefaltet und gemeinsam mit anderen Windeln zu transport- bzw. verkaufsfähigen Einheiten verpackt. Auch während dieser Prozesse sollte das Windelverschlussband in seinem Bereitstellungszustand verbleiben, damit die Windel, wenn sie dann genutzt werden soll, die Windelverschlussbänder in geeignetem Zustand, also in dem Bereitstellungszustand einem Nutzer präsentiert, so dass die Handhabe der Windeln möglichst ergonomisch und zuverlässig erfolgen kann, was insbesondere beispielsweise beim Wickeln von Kleinkindern oder auch unter Zeitdruck in Pflegesituationen einen durchaus bemerkenswerten Wert einer Windel darstellt.

Soll mithin ein Windelverschlussband, bei welchem ein Fastening-Bereich und ein Permanent-Bereich entlang der Länge des Trägerbands bzw. des Windelverschlussbands versetzt angeordnet sind, damit beispielsweise ein einfaches und ergonomisches Handling und ein angenehmer Tragekomfort gewährleistet werden kann, so erweist es sich als vorteilhaft, wenn die erste Komponente des zwei-komponentigen mechanischen Verschlusssystems in dem Arbeitszustand des Windelverschlussbands, also in dem Zustand, in welchem das Windelverschlussband seine eigentliche Hauptfunktion des öffenbaren Verschließens der Windeln erfüllen soll, eingenommen hat, in Richtung der Landezone, auf welchem die Komponenten beim Verschließen landen und mit welcher diese den zwei-komponentigen mechanischen Verschluss bilden soll, und damit in Richtung auf die Windelaußenseite bzw. in Richtung auf des Körpers des Trägers ausgerichtet ist.

Unter dieser Grundannahme des Arbeitszustandes ergibt sich, dass es von Vorteil ist, wenn das Windelverschlussband in den Bereitstellungszustand einen Windelrand der Windel umgreifend angeordnet ist, so dass eine unkontrollierte Bewegung des Windelverschlussbands während der Produktion, welche an sich schon durch die versetzte Anordnung des Fastening-Bereichs zu dem Permanent-Bereich auftreten könnte, möglichst kontrolliert werden kann. Durch die umgreifende Anordnung in den Bereitstellungszustand wird zunächst einmal unabhängig von allen übrigen Maßgaben eine mögliche Angriffsfläche für etwaige Kräfte, die zu unkontrollierten Bewegungen führen können, minimiert. Das Windelverschlussband wird dann weiter stabilisiert, wenn es in dem Bereitstellungszustand eine Verbindung des Fastening-Bereichs zu einem entsprechenden Bereich der Windel eingeht, was, da es sich um ein mechanisches Windelverschlussband handelt, vorzugsweise durch einen entsprechenden textilen Oberflächenbereich realisiert sein kann. Entsprechend der vorstehenden Vorgabe hinsichtlich des Arbeitszustandes folg, dass der textile Oberflächenbereich dann vorzugsweise an der Windelinnenseite, also an der dem Körper eines etwaigen Trägers zugewandten Seite angeordnet ist, was zudem den Vorteil hat, dass dieser textile Oberflächenbereich dann eine entsprechend angenehme Haptik aufweist, wenn er mit dem Körper des Windelträgers in Kontakt kommen sollte. Je nach konkreter Umsetzung kann beispielsweise die Innenseite des Windelkörpers entsprechend textil ausgebildet sein, um insbesondere auch flüssigkeitsdurchlässig zu sein und somit einen entsprechenden textilen Oberflächenbereich bereitstellen, mit welchem der Fastening-Bereich den Windelrand umgreifend in dem Bereitstellungszustand eine Verbindung eingehen kann. Ebenso ist es denkbar, dass ein entsprechender textiler Oberflächenbereich ergänzend bzw. nur für eine Befestigung des Fastening-Bereichs in der Bereitstellungsposition an der Windelinnenseite angebracht sein kann.

Je nach konkreter Ausgestaltung des Verschlusssystems der Windel und der Art, wie das Windelverschlussband einem Nutzer in der Bereitstellungsposition präsentiert werden soll, ist es jedoch auch denkbar, dass der textile Oberflächenbereich an der Windelaußenseite angeordnet sein kann, um dann dort den Fastening-Bereich in dem Bereitstellungszustand lösbar mit der Windel zu verbinden.

Es hat sich herausgestellt, dass insbesondere in dem Bereitstellungszustand, in welchem die Windel gerade während der Produktion eine möglichst stabile Verbindung auch des Fastening-Bereichs zu den textilen Oberflächenbereich bereitstellen soll, die Anforderungen an den textilen Oberflächenbereich verhältnismäßig speziell sind, da dieser an sich zunächst anderen Randbedingungen genügen soll. Dieses ist insbesondere ein angenehmes Hautgefühl bzw. eine angenehme Haptik, da diese Bereiche mit dem Körper in Kontakt treten können. Auch können eventuell entsprechende Eigenschaften, welche für die Innenschicht des Windelkörpers von Bedeutung sind, auch in dem textilen Oberflächenbereich, der mit dem Fastening-Bereich eine Verbindung in den Bereitstellungszustand eingehen soll, in Bezug auf die Verbindungsstärke nachteilig sein.

Dementsprechend erweist sich eine klebend wirksame Niederhaltefunkton von Vorteil, wie diese durch offene Bereiche einer Klebstoffschicht bereitgestellt werden kann, welche ohnehin an dem Trägerband bereits zum Einsatz kommt und der Befestigung der ersten Komponente der zwei-komponentigen mechanischen Verschlusssystems dient. Dieses ermöglicht eine entsprechend kostengünstige Herstellung, da keine separate Klebstoffschicht für diesen Zweck vorgesehen sein muss, was fertigungstechnisch beispielsweise auch hinsichtlich der Fertigungstoleranzen zu Problemen führen kann.

Dementsprechend kann - zunächst unabhängig von dem konkreten Merkmalen der vorliegenden Erfindungen jede Art von Klebstoff umfassenden, der geeignet ist, eine bzw. die erste Komponente des zwei-komponentigen mechanischen Verschlusssystems in ausreichendem Maße an dem Trägerband zu fixieren und andererseits in offenen Bereichen eine klebend wirksame Niederhaltefunktion zumindest in dem Bereitstellungszustand auszuüben. Je nach konkreter Umsetzung kann die klebend wirksame Niederhaltefunktion gegebenenfalls auch in dem Arbeitszustand ergänzend zu der Verbindungsstärke des mechanischen Verschlusses, mit welchem dann die Windel verschlossen wird, beitragen.

Wie bereits vorstehend dargelegt, nutzt die vorliegende Erfindung unter anderem einen Klebstoff für die Klebstoffschicht, welche einerseits die Komponente des zwei-komponentigen mechanischen Verschlusssystems an dem Trägerband klebend befestigen und andererseits ergänzend hierzu einen offenen Bereich zur Ausübung der Niederhaltefunktion aufweisen kann, welcher entsprechend des Dahlquist-Kriteriums kein klassischer Haftklebstoff ist, da das Dahlquist-Kriterium definiert, dass Klebstoffe mit einem Speichermodul *G'* von weniger als 3 10⁵ Pa, das bei Frequenzen von einem 1 Hz und bei Raumtemperatur bzw. bei 25 °C gemessen wird, als klebrig definiert werden, wie dieses insbesondere in der WO 2016 / 209 651 A1 zusammenfassend mit weiteren Nachweisen auf die US 6 884 504 bzw. auf das "Handbook of Pressure Sensitive Adhesive Technology" (2nd ed. (1989), S. 172 bis 176 mit der Aussage "Substanzen mit einem Speichermodul kleiner oder gleich des genannten Wertes würden als klebrig angesehen werden, wie dieses durch das Dahlquist-Kriterium definiert ist") erläutert wird. Es wird also vorliegend ein definitionsgemäß nicht klassischer Haftklebstoff, der eine Glasübergangstemperatur von wenigstens 23 °C aufweist, zur Ausübung der klebend wirksamen Niederhaltefunktion genutzt.

Im Konkreten soll das Speichermodul *G'* mehr als 3,3 10⁵ Pa bei 25 °C aufweisen, wobei die Klebstoffschicht vorzugsweise sogar ein Speichermodul *G'* von mehr als 3,6 10⁵ Pa, insbesondere von mehr als 4 10⁵ Pa aufweisen kann, um im Zusammenspiel mit einer Glasübergangstemperatur von wenigstens 23 °C noch die klebend wirksame Niederhaltefunktion auszuüben und darüber hinaus zu gewährleisten, dass bei ausreichend stabiler Befestigung der Komponente des zwei-komponentigen mechanischen Verschlusssystems nach dem Übergang vom dem Bereitstellungszustand in den Arbeitszustand eine ausreichende Verschlussfähigkeit gewährleistet werden kann.

Auch die Messungen des Speichermoduls *G'*, welche oberhalb des Dahlquist-Kriteriums liegen, erfolgen entsprechend den vorgenannten Rahmenbedingungen vorzugsweise bei einer Frequenz von 1 Hz und bei Raumtemperatur bzw. bei 25 °C.

Vorzugsweise weist die Klebstoffschicht ein Speichermodul *G'* von weniger als 7,0 10⁵ Pa, insbesondere von weniger als 6,0 10⁵ Pa auf, da sich herausgestellt hat, dass oberhalb dieser Werte eine ausreichende Klebekraft selbst bei im Übrigen optimalen Rahmenbedingungen, wie optimierten Breiten der offenen Bereiche der Klebstoffschicht in Abstimmung zu dem gewählten Material für die erste Komponente des zwei-komponentigen mechanischen Verschlusssystems, nicht mehr mit ausreichender Zuverlässigkeit gewährleistet werden kann.

Auch die verhältnismäßig hoch gewählte Glasübergangstemperatur spricht an sich gegen die Verwendung des entsprechenden Klebstoffes mit einer derartigen Glasübergangstemperatur als Klebstoffe zum Ausüben einer klebend wirksamen Niederhaltefunktion. Andererseits entsprechen die Umgebungsbedingungen während des Produktionsprozesses, bei welchem das Windelverschlussband an der Windel angebracht, um den Windelrand herumgefaltet und in dem Bereitstellungszustand bereitgestellt wird, nicht einer Raumtemperatur sondern liegen gegebenenfalls höher, was insbesondere auch der maschinellen Fertigung und den hohen Durchlaufgeschwindigkeiten geschuldet ist. Es wird davon ausgegangen, dass dementsprechend die Klebstoffschicht während der Produktion oberhalb Glasübergangstemperatur temperiert sein sollte, wobei dann ein Absinken der Übergangstemperatur, nachdem der Bereitstellungszustand eingenommen wurde, die Gefahr eines Auslösens von Fasern beim Übergang vom Bereitstellungszustand in den Arbeitszustand zu minimieren scheint. Insofern kann es sogar vorteilhaft sein, wenn die Klebstoffschicht eine Glasübergangstemperatur wenigstens 24,0 °C, insbesondere sogar von wenigstens 25,0 °C, aufweist.

Andererseits hat sich herausgestellt, dass ein Klebstoff mit einer zu hohen Glasübergangstemperatur dem komplexen Anforderungsbild, welches an diesen gestellt wird, nicht mehr zuverlässig genügen kann, auch wenn die übrigen Rahmenbedingungen, wie beispielsweise die konkrete Auswahl der ersten Komponente des zwei-komponentigen mechanischen Verschlusssystems, der Materialwahl und Oberflächengestaltung des Trägerbands sowie der Flächenanteil der offenen Bereiche optimiert sind. Insofern ist es von Vorteil, wenn die Klebstoffschicht eine Glasübergangstemperatur von weniger als 28,0 °C vorzugsweise sogar von weniger 27,5 °C, aufweist.

Insoweit kann insbesondere die WO 2016/140944 A1 diesbezüglich keinerlei erläuternde Hinweise liefern. In dieser Druckschrift wird zwar die Einordnung des dort genutzten Klebstoffs hinsichtlich des Dahlquist-Kriteriums vorgenommen, allerdings liegt das dort genutzte Fenster in einem deutlich anderen Bereich al das des vorliegend erläuterten Klebstoffs. Darüber hinaus wird in dieser Druckschrift die Einordnung des dort offenbarten Klebstoffs als pressuresensitive bzw. als Haftklebstoff oder als elastomerer Klebstoff ausgeschlossen.

Kumulativ bzw. alternativ zu dem vorstehend diskutierten, auf das Dahlquist-Kriterium und die Glasübergangstemperatur gerichteten Merkmalskombinationen wurde bereits eingangs die Kombination bestimmter Grenzwerte der Sofortklebekraft und der Klebkraft als kennzeichnende Merkmale für die Klebstoffschicht erläutert.

Diesbezüglich sei festgehalten, dass die Sofortklebekraft auch als Anfangshaftung (loop-tack) eines Selbstklebematerials bezeichnet werden kann und ein Maß für das "Anfassen" oder den "Anfangs-Tack" von Haftmaterialien darstellt.

Die Anfangshaftung bzw. die Sofortklebekraft eines Selbstklebematerials kann insbesondere als die Kraft gemessen werden, welche benötigt wird, eine Schlaufe des Materials mit der Klebstoffseite außen, die mit einer Standardprüffläche in Berührung gebracht wird, mit einer vorgegebenen Geschwindigkeit zu trennen.

Letztlich stehen im Prinzip sehr unterschiedliche Messmethodiken zur Verfügung, um die Sofortklebekraft bzw. die Anfangshaftung zu quantifizieren bzw. zu messen. In vorliegendem Zusammenhang hat es sich als besonders vorteilhaft erwiesen, wenn die Sofortklebekraft durch eine loop-tack-Prüfung quantifiziert wird, u.a. wie sie vorstehend definiert ist. Insbesondere kann die loop-tack-Prüfung nach der FINAT Testmethode FTM9 - siehe beispielsweise FINAT Technisches Handbuch, 6. Ausgabe, Den Haag 2001 - bzw. nach einer hieran angelehnten Testmethode erfolgen.

Für die loop-tack-Prüfung kann insbesondere ein Zugfestigkeitsmessgerät oder ein ähnliches Prüfgerät mit umkehrbarer senkrechter Klemmenbewegung von 300 mm/min ± 2 % dienen. Die Kraftmessung sollte bis zu 20 N mit einer Genauigkeit von ± 2 % möglich sein.

Des Weiteren kann für die loop-tack-Prüfung eine ebene Platte als Testplatte zur Anwendung kommen. Vorzugsweise weist diese Platte die Maße Länge: 25 ± 0,5 mm × Breite: 30 ± 2,0 mm auf, wobei die 25 mm letztlich als Normbreite für derartige Messungen vorteilhaft genutzt werden, da dieses eine in vorliegendem Zusammenhang eine übliche Breite, insbesondere auch für dann die Windelverschlussbänder, darstellt. In diesem Zusammenhang sei erwähnt, dass selbstverständlich auch andere Breite genutzt werden können, was dann zu entsprechenden Umrechnungen führen muss. Die Breite von 30 mm übersteigt dann die Breite der Prüflinge, welche insbesondere 25 mm breit gewählt werden, so dass ausreichend Spiel für die Ausrichtung der Schlaufe besteht, während die 25 mm die Länge darstellen, über welche dann der klebende Kontakt bei der loop-tack-Prüfung geschlossen und wieder geöffnet wird. Die Breite der Testplatte kann gegebenenfalls auch größer und insbesondere bis zu 50 mm oder sogar darüber gewählt werden, wodurch der Messweg bzw. der Trennweg entsprechend verlängert wird.

Die Testplatte sollte ausreichend stark bzw. dick sein, damit sie sich während der Messung nicht verformt oder zu stark schwingt. Je nach konkretem Material der Testplatte, können hier unterschiedliche Testplattendicken ausreichen, solange Durchbiegungen bzw. Schwingungen im ausreichenden Maße reduziert sind.

Entsprechend der FTM9 sollte als Testplatte eine Platte aus Kristallspiegelglas (float glas) beispielsweise der Fa. Rocholl zur Anwendung kommen, welche dann zumindest 3,0 mm dick sein sollte. In alternativen Testmethoden können insbesondere AFERA-Stahl oder PE-, PP- oder PVC- Platten, beispielsweise der Fa. Rocholl zur Anwendung kommen, wenn eine gezielte Prüfung gegen entsprechende Materialien erfolgen soll. Gegebenenfalls können auch mit verschiedenen Substraten geklebte Testplatten genutzt werden, wenn besondere Materialkonstellationen im Detail überprüft werden sollen.

Als Testplatten können insbesondere PE-Testplatten unter dem Handelsnamen PE-HWST bzw. PP-Testplatten unter dem Handelsnamen PP-DWST der Fa. Rocholl zum Einsatz kommen. Bei diesen Testplatten hat sich herausgestellt, dass eine Stärke von 2 mm für die durchgeführten Tests zu ausreichend reproduzierbaren Ergebnissen führt.

An der Unterseite der Testplatten ist vorzugsweise eine Anordnung vorgesehen, die es ermöglicht, dass die Testplatte jeweils in das Zugfestigkeitsmessgerät oder das im Übrigen genutzte Prüfgerät eingesetzt werden kann. Je nach konkreter Umsetzung, kann diesbezüglich ein Metallstift ausreichen, der in eine untere Klemme des Prüfgeräts bzw. des Zugfestigkeitsmessgeräts passt.

Bei der konkreten Umsetzung einer Prüfung werden Teststreifen aus einem repräsentativen Muster des Materials geschnitten. Die Streifen sind, wie bereits vorstehend erläutert, vorzugsweise 25 mm breit, wobei diesbezüglich auch Abweichungen vorgesehen sein können, insoweit dann entsprechende Umrechnungsfaktoren berücksichtigt werden.

Die Schnitte sollten sauber und gerade sein, um möglichst Verfälschungen der Messergebnisse zu vermeiden.

Vorzugsweise werden jeweils 5 Teststreifen aus einem repräsentativen Muster des Materials geschnitten, um auf diese Weise mehrere Messungen durchführen zu können und eine Fehlerkontrolle zu ermöglichen.

Als Länge des Teststreifens haben sich eine Mindestlänge von 175 mm, insbesondere eine Mindestlänge von 250 mm, bzw. eine Länge von unter 300 mm als vorteilhaft erwiesen. Gegebenenfalls werden an den Enden 20 mm oder mehr abgeklebt, um insbesondere eine Kontamination von Klemmbacken der verwendeten Prüfgeräte bzw. Zugfestigkeitsmessgeräte zu vermeiden bzw. zu minimieren.

Unmittelbar vor der Prüfung wird ein etwaiges Trennmaterial von den Teststreifen entfernt. Falls die Teststreifen ohne Trennmaterial bzw. Abdeckpapier vorliegen, wären diese vor dem Schneiden auf Silikonpapier oder auf ähnlichem Trennmaterial aufzukleben und erst dann geschnitten. Eine Lagerung auf derartig hinzugefügte Trennmaterialien sollte jedoch nicht erfolgen, um Verfälschungen zu vermeiden.

Die Messung erfolgt vorzugsweise bei 23 ± 2 °C und 50 ± 5 % relativer Luftfeuchtigkeit. Hierbei sollten die repräsentativen Muster bzw. Teststreifen vor der Prüfung mindestens 4 Stunden klimatisiert sein. Sollte das repräsentative Muster in Form von Rollenware vorliegen sollte die Klimatisierungszeit mindestens 24 Stunden betragen.

Die Testplatten werden vorzugsweise vor der Prüfung sorgfältig gereinigt, insbesondere dass keine Spuren von Klebstoffen, Fett, Silikon, Feuchtigkeit oder anderen Verunreinigungen zurückbleiben. Je nach Material der Testplatten, kann beispielsweise Spezialbenzin 60/95 diesbezüglich genutzt werden.

Ggf. können auch andere geeignete Reinigungsmethoden, wie beispielsweise Ultraschallreinigungen, zur Anwendung kommen, um Verunreinigungen gründlich zu entfernen.

Nach der Reinigung sollten die Testplatten mindestens 10 Minuten abdampfen und klimatisieren. Gereinigte Platten sollten nur an den Kanten angefasst werden, insbesondere um eine nachträglich Re-Kontamination zu vermeiden.

Vor der Prüfung sollten die Testplatten auch auf etwaige Kratzer oder Beschädigungen geprüft werden, damit möglichst unverfälschte Messergebnisse gewonnen werden können. Auch eine Lagerung der Testplatten erfolgt vorzugsweise derart, dass die Prüfoberflächen nicht verkratzen oder beschädigt werden.

Zur Reinigung können auch Diacetonalkohol technischer Qualität oder besser, Methylethylketon (MEK), Aceton, Methanol 95 %-ig, n-Heptan oder Ethylacetat zur Anwendung kommen.

Das zur Reinigung eingesetzte Material sollte saugfähig sein, beispielsweise Watte, Vliesmaterial oder Textilien. Diese Materialien sollten während des Gebrauchs nicht fasern, Lösungsmittel aufnehmen und keine Bestandteile enthalten, welche in den oben genannten Lösungsmitteln löslich sind. Vorzugsweise werden zum Reinigen frische Materialien eingesetzt, um ein Kontaminationsrisiko und einen Verschleiß dieser Materialien zu minimieren.

Für die Reinigung wird die Prüfplatte mit dem entsprechenden Lösungsmittel benetzt und mit frischem Reinigungsmaterial trockengewischt. Vorzugsweise kann die Reinigung mit Lösungsmittel dreimal durchgeführt werden, wobei gegebenenfalls auch das Lösungsmittel variiert wird. Je nach konkreter Wahl der Testplatte, kann insbesondere als letzten Reinigungsschritt eine Reinigung mit MEK oder Aceton erfolgen. Insbesondere bei der Reinigung von Kunststoffplatten, beispielsweise aus PE, PP, PVC usw., hat sich eine Reinigung mit Spezialbenzin 60/90 als vorteilhaft herausgestellt.

Vor der Verwendung sollten die gereinigten Prüfplatten ebenfalls 4 Stunden klimatisieren.

Unmittelbar vor der Prüfung wird das Trennmaterial von dem jeweiligen Teststreifen bzw. das ergänzend zum Schneiden benutzte Silikonpapier von dem Teststreifen entfernt. Die beiden Enden des Teststreifens werden so zusammengelegt, dass sich eine Schlaufe mit nach außen gerichtete Klebstoffseite bildet.

Die beiden Enden der Schlaufen werden bei einer ausreichenden Strecke in der oberen Klemme des Zugfestigkeitsmessgerätes bzw. des Prüfgeräts befestigt, so dass Schlaufe senkrecht nach unten hängt. Die eingespannte Strecke kann insbesondere zwischen 10 mm und 20 mm betragen.

Dann wird das Prüfgerät gestartet und die Schlaufe - nach Möglichkeit sofort - auf die waagerecht befestigte Testplatte mit einer Geschwindigkeit von 300 mm pro Minute gefahren, bis eine Kontaktfläche in der Länge der Testplatte entsteht. Sollte der Teststreifen die Testplatte nicht über die volle Länge abdecken, sollte Klemmabstand entsprechend angepasst werden. Alternativ hierzu kann gegebenenfalls auch die Länge des Teststreifens entsprechend erhöht werden.

Sobald die Testplatte vollständig bedeckt ist, wird Prüfgerät sofort in Gegenrichtung umgeschaltet und die Schlaufe wieder nach oben gezogen. Auch hier wird eine Geschwindigkeit von 300 mm pro Minute vorzugsweise eingehalten, so dass sich Schlaufe und Testplatte mit 300 mm pro Minute trennen.

Es ist darauf zu achten, dass die Zeit für die Richtungsumkehr so kurz wie möglich gehalten wird.

Als wesentlicher Messwert kann insbesondere der Höchstwert der Kraft, welcher zum Trennen der Schlaufe von der Testplatte benötigt wird, notiert werden.

Vorzugsweise werden zumindest 5 Teststreifen getestet und ein entsprechender Mittelwert gebildet.

Insbesondere durch eine Sofortklebekraft von 1,0 N/(25 mm) oder mehr kann sichergestellt werden, dass auch bei sehr hohen Maschinengeschwindigkeiten das Windelverschlussband in dem Fastening-Bereich ausreichend betriebssicher klebend niedergehalten wird. Insbesondere kann die Sofortklebekraft der Klebstoffschicht über 1,5 N/(25 mm) bzw. sogar über 1,8 N/(25 mm) liegen, wobei diese Werte vorzugsweise gegen Polyethylen gemessen werden können. Je nach konkreten Verhältnissen kann die Sofortklebekraft der Klebstoffschicht sogar über 2,0 N/(25 mm), insbesondere über 2,5 N/(25 mm), liegen, wobei dann die Messungen vorzugsweise gegen Polypropylen erfolgen.

Die Gefahr von Faserausrissen, insbesondere auch bei einem Öffnen und Wiederverschließen der Windel, was gegebenenfalls sehr schnell durch hastige Bewegungen erfolgen kann, kann minimiert werden, wenn die Sofortklebekraft unter 5,0 N/(25 mm), insbesondere unter 6,0 N/(25 mm) und besonders vorzugsweise unter 5,0 N/(25 mm), liegt. Je nach konkreten Verhältnissen kann die Sofortklebekraft sogar unter 4,0 N/(25 mm) bzw. unter 3,0 N/(25 mm) liegen.

Hierbei hat es sich in konkreter Umsetzung als vorteilhaft erwiesen, wenn die Sofortklebekraft unter 6,0 N/(25 mm), insbesondere unter 5,0 N/(25 mm), liegt, wenn diese Messungen vorzugsweise gegen Polypropylen erfolgen. Alternativ bzw. kumulativ kann die Sofortklebekraft unter 4,0 N/(25 mm), insbesondere unter 3,0 N/(25 mm), liegen, wenn die Messungen vorzugsweise gegen Polyethylen erfolgen.

Wie bereits weit vorstehend erläutert, kann die Klebstoffschicht eine Klebkraft zwischen 10,0 N/(25 mm) und 25,0 N/(25 mm) aufweisen, um ergänzend zu etwaigen Detailgrenzen der Sofortklebekraft sicherzustellen, dass bei ausreichender Niederhaltefunktion in dem Bereitstellungszustand und bei ausreichend stabiler Befestigung der Komponente des zwei-komponentigen mechanischen Verschlusssystems nach dem Übergang von dem Bereitstellungszustand in den Arbeitszustand eine ausreichende Verschlussfähigkeit gewährleistet werden kann.

Hierbei kann insbesondere die Klebkraft als die Kraft bezeichnet werden, welche erforderlich ist, ein Selbstklebematerial, dass unter bestimmten Bedingungen auf eine Standardtestplatte geklebt wurde, von dieser Platte mit einem bestimmten Abzugswinkel und einer bestimmten Geschwindigkeit wieder abzulösen. Insoweit kann als Klebkraft die Permanenthaftung bzw. die Ablösbarkeit eines Haftmaterials bezeichnet werden.

Zur Ermittlung der die Klebkraft kann insbesondere eine 180°-peel-adhesion-Prüfung durchgeführt werden, durch welche in der Regel in Bezug auf die Klebkraft reproduzierbare Messergebnisse zu erwarten sind.

Bei einer 180°-peel-adhesion-Prüfung wird hierbei ein gewählter Teststreifen vorzugsweise von einer Testplatte in einem Winkel von 180° abgezogen, wobei hierbei dann die Kräfte gemessen und entsprechend ausgewertet werden können.

Insbesondere kann die 180°-peel-adhesion-Prüfung nach der FINAT-Testmethode FTM 1 oder nach einer an die FTM 1 angelehnten Testmethode erfolgen, so dass sich standardisierte und vergleichbare Messergebnisse gewinnen lassen.

Je nach konkreten Testinteresse kann insbesondere bei 20 Minuten sowie 24 Stunden nach dem Aufkleben eine entsprechende Prüfmessung durchgeführt werden, wobei in diesem Zusammenhang der Vorgang nach 24 Stunden nach dem Aufkleben auch als Enthaftung bezeichnet wird.

Je nach konkret genutztem Prüfverfahren kann auch für diese Prüfung ein Zugfestigkeitsmessgerät oder ein entsprechendes Prüfungsgerät zur Anwendung kommen, dass einen Verbund zweier Materialien mit einem Abzugswinkel von 180° und einer Klemmgeschwindigkeit von 300 mm pro Minute mit einer Genauigkeit von ± 2 % trennen kann.

Als Testplatten kommen insbesondere die bereits in Bezug auf die loop-tack-Prüfung bereits erläuterten Testplatten in Frage, insoweit diese dazu ausgebildet sind, für eine 180°-Prüfung in ein entsprechendes Prüfgerät, insbesondere in ein Zugfestigkeitsmessgerät, eingesetzt zu werden.

Vorzugsweise wird eine FINAT-Standard-Anpressrolle verwendet, um den zu prüfenden Verbund aus Testplatte und Teststreifen bereitstellen zu können, wie nachfolgend noch erläutert wird.

Die Teststreifen werden aus einem repräsentativen Muster des Materials geschnitten. Sie sind vorzugsweise 25 mm breit und haben in Laufrichtung eine Mindestlänge von 175 mm bzw. 250 mm. Der Schnitt sollte sauber und gerade sein. Sollte das Muster ohne Trennmaterial vorliegen, so empfiehlt es sich, dieses vor dem Schneiden bzw. Trennen auf Silikonpapier aufzukleben, wobei einer Lagerung auf einem hierzu ergänzten Silikonpapier vermieden werden sollte.

Als Testbedingungen werden 23 ± 2 °C und 50 ± 5 % relativer Luftfeuchtigkeit gewählt, wobei auch bei dieser Prüfung sowohl die Teststreifen als auch die Testplatten nach der Reinigung mindestens 4 Stunden entsprechend klimatisiert werden sollen. Sollten die Teststreifen aus einem aufgewickelten bzw. aufgerollten Material stammen bzw. derart gelagert sein, so empfiehlt sich eine Klimatisierung von wenigstens 24 Stunden.

Zunächst wird für den Prüfvorgang das Trennmaterial von dem Teststreifen abgezogen. Gegebenenfalls wird dementsprechend auch das Silikonpapier abgezogen.

Anschließend wird der Teststreifen vorzugsweise mit leichtem Fingerdruck mit seiner Klebstoffseite auf die vorbereitete Testplatte geklebt. Vorzugsweise wird im Anschluss die FINAT-Standard-Anpressrolle mit einer Geschwindigkeit von ca. 10 mm pro Sekunde zweimal in beide Richtungen über den aufgeklebten Streifen gerollt, um einen guten und definierten Kontakt zwischen dem Klebstoff und der Oberfläche der Testplatte herzustellen.

Die auf diese Weise vorbereiteten Testmuster bleiben nach dem Aufkleben 20 Minuten liegen und werden dann, wie nachfolgend im Detail erläutert wird, geprüft. Ein zweiter Satz kann auf dieselbe Weise vorbereitet und nach 24 Stunden geprüft werden, wenn dieses hinsichtlich des Testergebnisses entsprechend gewünscht ist.

Zu Beginn des eigentlichen Prüfvorgangs wird die Testplatte derart in dem Prüfgerät bzw. in dem Zugfestigkeitsmessgerät befestigt, dass der Teststreifen einen Abzugswinkel von 180° Grad erhält. Die Klemmengeschwindigkeit wird auf 300 mm pro Minute eingestellt. Vorzugsweise wird während der Prüfung die Kraft mindestens fünfmal in Abständen von 10 mm im mittleren Bereich des Streifes abgelesen.

Aus den Messungen kann dann ein entsprechender Durchschnittswert für die Kraft ermittelt werden.

Entsprechend der Breite der Teststreifen kann die Klebkraft dann als Durchschnittswert der geprüften Teststreifen in N/(25 mm) für 20 Minuten bzw. 24 Stunden Kontaktzeit zwischen Aufkleben und Messung angegeben werden.

Insbesondere kann die Klebkraft 10,0 N/(25 mm) und mehr betragen, um auf diese Weise dauerhaft bzw. während der üblichen Lebensdauer von Windeln ausreichend stabile Befestigung der Komponente des zwei-komponentigen mechanischen Verschlusssystems zu gewährleisten. Je nach konkreter Umsetzung kann durch eine entsprechende Klebkraft auch eine gute Niederhaltefunktion im Arbeitszustand des Windelverschlussbandes gewährleistet werden. Die entsprechenden Vorteile ergeben sich insbesondere, wenn die Klebkraft über 12,0 N/(25 mm), insbesondere über 13,0 N/(25 mm), beträgt, wobei letztere Messung vorzugsweise gegen Polyethylen gemessen werden können. Gegebenenfalls kann die Klebkraft insbesondere auch über 15,0 N/(25 mm), vorzugsweise über 17,0 N/(25 mm), betragen, wobei diese Messungen gegen vorzugsweise Polypropylen vorgenommen werden.

Da produzierte und verpackte Windeln gegebenenfalls über längere Zeit gelagert werden, ist es von Vorteil, wenn die Klebkraft 25,0 N/(25 mm) oder weniger aufweist. Auf diese Weise können insbesondere Faserausrisse beim Übergang von dem Bereitstellungszustand in den Arbeitszustand auf ein Minimum reduziert werden. Dieses gilt insbesondere, wenn die Klebkraft unter 23,0 N/(25 mm) liegt. Je nach konkreten Verhältnissen kann die Klebkraft insbesondere unter 20,0 N/(25 mm), vorzugsweise unter 18,0 N/(25 mm), liegen, um entsprechende Vorteile sicherzustellen. Hierbei kann die Klebkraft vorzugsweise gegen Polypropylen gemessen werden und vorzugsweise unter 25,0 N/(25 mm), insbesondere unter 23,0 N/(25 mm), liegen. Alternativ bzw. kumulativ kann die Klebkraft gegen Polyethylen gemessen werden, wobei dann die Klebkraft insbesondere unter 20,0 N/(25 mm), vorzugsweise unter 18,0 N/(25 mm), liegen kann.

Wie bereits eingangs erläutert, ist es vorteilhaft, wenn die Klebstoffschicht einem Öffnen der Verbindung in dem Fastening-Bereich von dem Bereitstellungszustand ausgehend in den Arbeitszustand eine peel-Mindestkraft von wenigstens 0,6 N/(25 mm) entgegensetzt und bei dem Öffnen weniger als 20 Fasern pro 0,125 cm² aus dem textilen Oberflächenbereich ausreißt.

Unmittelbar nachvollziehbar ist, dass es entsprechend vorteilhafter ist, wenn die Klebstoffschicht bei dem Öffnen weniger als 17 Fasern, insbesondere weniger als 15 Fasern, pro 0,125 cm² aus dem textilen Oberflächenbereich ausreicht, da dann das Maß der Kontamination des Fastening-Bereichs, insbesondere der Komponente des zwei-komponentigen Verschlusssystems und gegebenenfalls auch noch offener Bereiche der Klebstoffschicht, welche auch während des Schließens der Windel eine Niederhaltefunktion aufbringen sollen, noch geringer ist.

Zur Kontrolle der Zahl der ausgerissenen Fasern erfolgt das Öffnen der Verbindung zwischen dem Fastening-Bereich einerseits und dem textilen Oberflächenbereich andererseits vorzugsweise nach dem Entnehmen der Windel aus einem Verpackungsverbund, sodass diesbezüglich letztlich das Verhalten eines Nutzers nachgestellt wird.

In vorliegendem Zusammenhang sei erwähnt, dass die ausgerissenen Fasern sowohl ganze Fasern als auch Bruchstücke der Fasern des textilen Oberflächenbereichs sein können. Insbesondere wenn komplexe Fasern, wie beispielsweise Garne, zum Einsatz kommen, können auch Einzelfasern aus dem komplexen Verbund entsprechend ausgerissen werden, so dass auch diese als ausgerissene Fasern bezeichnet werden.

Zur Bestimmung peel-Mindestkraft kann insbesondere ein 2*90°-Test durchgeführt werden. Bei einem solchen Test können unter kontrollierten Bedingungen der textile Oberflächenbereich sowie der Fastening-Bereich jeweils mit einer in einem Winkel von 90° zu der Verbindungsfläche zwischen dem Fastening-Bereich und dem textilen Oberflächenbereich ausgerichteten Kraftrichtung voneinander entfernt werden, was dann ein objektives Maß für die Stärke der Verbindung darstellen kann.

Auch für diesen Test kann es vorteilhaft sein, eine Windel unmittelbar aus der Verpackungseinheit zu entnehmen, den textilen Oberflächenbereich, welcher von dem Windelverschlussband umgriffen wird, herauszuschneiden und das dem Permanent-Bereichs des Windelverschlussbandes abgewandte Ende, beispielsweise den Fingerlift, einerseits sowie einen kleinen Überstand des textilen Oberflächenbereichs im Bereich dieses Endes des Windelverschlussbandes bzw. in der Nähe des Permanent-Bereichs des Windelverschlussbandes andererseits in einem Zugfestigkeitsmessgerät oder einem entsprechenden Prüfgerät, das einen Verbund mit einem Abzugswinkel von 180° und einer Klemmengeschwindigkeit von 300 mm pro Minute mit einer Genauigkeit von ± 2 % trennen kann, einzuspannen.

Vor der Entnahme aus der Verpackungseinheit empfiehlt es sich, die Verpackungseinheit als Ganzes über mindestens 24 Stunden auf 23 ± 2 °C und 50 ± 5 % relative Luftfeuchtigkeit zu klimatisieren, wobei es dementsprechend von Vorteil ist, wenn auch der 2*90°-Test unter entsprechenden klimatischen Bedingungen durchgeführt wird.

Um die Gefahr einer Verfälschung es Messergebnisses zu minimieren, empfiehlt es sich, das Windelverschlussband selbst nicht zu durchtrennen, auch wenn dieses breiter als 25 mm ist. Die Breite kann jedoch vor bzw. nach der Messung ermittelt werden, um das Messergebnis dann entsprechend dieser Breite auf 25 mm Breite zu normieren.

Je nach konkreter Windel, welche einer entsprechende Messung unterzogen werden soll, kann beispielsweise der Fingerlift oder der textile Oberflächenbereich mit einem Verstärkungsband versehen werden, um ein Einspannen in die Klemmen des Zugfestigkeitsmessgerätes bzw. des entsprechendes Prüfgeräts zu erleichtern bzw. zu ermöglichen. Insbesondere kann hierdurch auch eine entsprechend Verlängerung des Fingerlifts bzw. des textilen Oberflächenbereichs erfolgen, sodass eine ausreichende Einspannlänge gewährleistet werden kann.

In diesem Zusammenhang sei erwähnt, dass gegebenenfalls auch andere ergänzende Maßnahmen vorgesehen sein können, um eine sichere Befestigung der Teststreifen, der Baugruppen des Windelverschlussbandes bzw. der textilen Oberflächenbereiche in den jeweiligen Prüfgeräten zu gewährleisten.

Im Anschluss an ein Einspannen werden das Windelverschlussband und der textile Oberflächenbereich voneinander mit einer Abzugsgeschwindigkeit von 300 mm pro Minute abgezogen, bis lediglich die Verbindung durch den Permanentteil verbleibt.

Vorzugsweise erfolgt eine Auswertung über den gesamten Messweg, wobei durchaus eine Analyse des Messergebnisses innerhalb der ersten 20 %, insbesondere innerhalb der ersten 10 % und/oder innerhalb der letzten 20 %, insbesondere innerhalb der letzten 10 %, des Messweg erfolgen kann, ob in diesem Bereich nicht irgendwelche Artefakte das Messergebnis verfälschen, so dass diese Anfangs- bzw. Endbereiche der Messung ggf. ausgeschlossen werden können oder sollten. Aufgenommen wird die Kraft, wobei insbesondere Minimalkräfte und Maximalkräfte ausgewertet werden können.

Zur Überprüfung der Genauigkeit der Messergebnisse ist es von Vorteil, wenn mehrere Proben genommen werden können, wobei in der Regel je Windel zumindest zwei Proben genommen werden können, da in der Regel jeweils zwei Windelverschlussbänder an einer Windel zu finden sind. Da üblicherweise Windeln in größeren Verpackungseinheiten am Markt zu finden sind, können aus einer Verpackungseinheit auch stichprobenartig Windeln für entsprechende Messungen gewonnen werden.

Die Durchführung mehrerer Messungen ermöglicht dann eine Auswertung durch entsprechende Mittelwerte und Standardabweichungen.

Es ist insbesondere vorteilhaft, wenn bei dem 2*90°-Test ebenfalls weniger als 20 Fasern, insbesondere weniger als 17 Fasern, vorzugsweise weniger als 15 Fasern, aus dem textilen Oberflächenbereich ausgerissen werden. Unter den gegebenen Versuchsbedingungen ist es den Erfindern sogar gelungen, dass bei geeigneten Ausgestaltungen der Windelverschlussbänder in Bezug auf die textilen Oberflächen, bei derartigen Tests keine Fasern aus dem textilen Oberflächenbereich ausgerissen wurden.

Insbesondere kann die peel-Mindestkraft wenigstens 0,5 N/(25 mm), vorzugsweise wenigstens 0,7 N/(25 mm), geringer als die Maximalkraft, welche das zwei-komponentige mechanische Verschlusssystem dem Öffnen der Verbindung entgegensetzt, betragen. Diesbezüglich hat sich herausgestellt, dass in den meisten Fällen deutliche Schwankungen der peel-Kraft in Abhängigkeit von offenen Bereichen der Klebstoffschicht einerseits oder der ersten Komponente des zwei-komponentigen mechanischen Verschlusssystems andererseits, die jeweils mit dem textilen Oberflächenbereich in Kontakt sind, zu finden sind, wenn der Fastening-Bereich von dem textilen Oberflächenbereich durch bzw. während des 2*90°-Tests getrennt wird. Insoweit die peel-Mindestkraft der Klebstoffschicht zumindest ausreichend gering ist, wie durch den Grenzwert von 0,5 N/(25 mm), bzw. insbesondere 0,7 N/(25 mm), im Vergleich zu der Maximalkraft, welche das zwei bestimmte komponentige mechanische Verschlusssystem dem Öffnen der Verbindung entgegensetzt, erfolgt, die Gefahr, dass Fasern durch die Klebstoffschicht ausgerissen werden, entsprechend niedrig ist.

Vorzugsweise weist das Windelverschlussband eine Länge unter 100 mm, insbesondere unter 95 mm, und/oder über 30 mm, insbesondere über 35 mm, auf. Obgleich letztendlich nach unten keine wesentlichen Grenzen gesetzt sind, ist bei zu kleinen Windelverschlussbändern dann die Handhabe zu erschwerlich und die Gesamtverschlusskraft, welche ein zugehöriger Fastening-Bereich bereitstellen kann, zu gering, als dass derartige Windelverschlussbänder in der Praxis wirtschaftlich zum Einsatz kommen können. Bei längeren Windelverschlussbändern ist davon auszugehen, dass es entweder andere Verschlussmechanismen oder aber andere Niederhaltemaßnahmen gibt, um die Produktion und die Funktionsweise entsprechender Windeln sicherzustellen.

Bei der in der EP 0 974 326 B1 vorgeschlagenen Lösung ergeben sich in dem Bereitstellungszustand die vorstehend beschriebenen Problematiken nicht, da die in dieser Druckschrift vorgeschlagene Lösung eine Release-Band nutzt, auf welchem der offene Bereich der Klebstoffschicht in dem Bereitstellungszustand ruht, so dass an dieser Stelle die Gefahr einer Kontamination durch Faserausrisse nicht bestehen kann. Hieraus resultiert, dass in dieser Druckschrift auch andere Auswahlkriterien für den Klebstoff gewählt werden, als in vorliegendem Zusammenhang. Auch wird in dieser Druckschrift vorgeschlagen, nicht nur die Oberfläche des Release-Bands sondern auch die Fasern des Auftreffbauteils antihaftend beschichtet sind, um ein Ausreißen von Fasern zu vermeiden. Diesbezüglich wird vorliegend durch die geeignete Wahl des Klebstoffs insbesondere hinsichtlich seiner Sofortklebekraft, aber auch hinsichtlich der anderen Parameter ein gänzlich anderer Weg beschritten.

Während vorliegend die klebend wirksame Niederhaltefunktion insbesondere den Bereitzustellungszustand von Bedeutung erscheint, ist es denkbar, dass eine entsprechende Niederhaltefunktion auch im Arbeitszustand ergänzend zu einem mechanischen Verschlusssystem, welches dann die Außenschicht der Windel oder eine entsprechende Landezone, wie sie vorstehend bereits beschrieben wurde, einschließt, unterstützen kann. Auch hier ist eine Kontamination der offenen Bereiche der Klebeschicht und des mechanischen Verschlusssystems, insbesondere bei einem Öffnen und Wiederverschließen für etwaige Kontrollzwecke oder Sitzkorrekturen, nicht förderlich und mithin möglichst zu minimieren.

Vorzugsweise weist die Klebstoffschicht einen auf der den Permanent-Bereich zugewandten Seite der ersten Komponente des zwei-komponentigen mechanischen Verschlusssystems angeordneten offenen Bereich auf. Eine derartig angeordneter offener Bereich ermöglicht eine entsprechende Niederhaltefunktion an dieser Stelle, an welcher häufig ein Zwischenbereich, der zwischen dem Fastening-Bereich und dem Permanent-Bereich zu finden ist, anfängt, und welcher in der Regel keine klebenden Bereiche zur Windel hin aufweist. Insofern kann dieser offene Bereich gerade einen derartigen Zwischenbereich ergänzend niederhalten und so das Windelverschlussband zuverlässig in seinem Bereitstellungszustand gehalten werden kann.

Je nach konkreter Ausgestaltung kann die Klebstoffschicht zum Permanent-Bereich an diesen offenen Bereich enden, sodass dann der Zwischenbereich sich ohne weiteres anschließen kann. Alternativ ist es auch möglich, die Klebstoffschicht weiter in Richtung auf den Permanent-Bereich zulaufen zu lassen und in den Zwischenbereich die Klebstoffschicht entsprechend abzudecken bzw. zu kaschieren oder auf sonstige Weise zu inaktivieren. Gegebenenfalls kann die Klebstoffschicht sogar bis in den Permanent-Bereich reichen und, insoweit der Klebstoff bzw. die komplementäre Oberfläche der Windel geeignet abgestimmt werden kann, auch der permanenten Befestigung des Windelverschlussbands an der Windel dienen.

Vorzugweise ist der offene Bereich auf der dem Permanent-Bereich zugewandten Seite der ersten Komponente des zwei-komponentigen mechanischen Verschlusssystems mindestens 0,3 mm, insbesondere zumindest 0,4 mm, breit, sodass auf diese Weise eine ausreichend große Niederhaltefunktion gewährleistet werden kann.

Je nach konkreter Umsetzung und insbesondere auch in Abhängigkeit von dem textilen Oberflächenbereich bzw. in Abhängigkeit von der Art der ersten Komponente des zwei-komponentigen mechanischen Verschlusssystems kann der offene, auf den der Permanent-Bereich zugewandten Seite der ersten Komponente des zwei-komponentigen mechanischen Verschlusssystems angeordnete Bereich der Klebstoffschicht auch breiter vorgesehen sein.

Insbesondere ist es jedoch von Vorteil, wenn der offene Bereich der Klebstoffschicht, welche auf der dem Permanent-Bereich zugewandten Seite der ersten Komponente des zwei-komponentigen mechanischen Verschlusssystems angeordnet ist, nicht mehr als 3 mm, insbesondere nicht mehr als 2,6 mm, breit ist, was für übliche Windelverschlussbänder als eine ausreichende Breite angesehen werden kann, wobei mit zunehmender Breite letztlich auch das Risiko steigen könnte, dass diese offene Bereich zunehmend Fasern aus dem textilen Oberflächenbereich ausreißen könnte.

Unabhängig hiervon kann es von Vorteil sein, wenn sich der offene Bereich der Klebstoffschicht, der auf der Permanent-Bereich zugewandten Seite der ersten Komponente des zwei-komponentigen mechanischen Verschlusssystems angeordnet ist, nicht mehr als 60 %, vorzugsweise nicht mehr als 50 % des Abstands zwischen dem Fastening-Bereich und dem Permanent-Bereich, also der Längserstreckung des Zwischenbereichs, wenn denn ein solcher definiert ist, erstreckt.

Durch eine entsprechende Begrenzung dieser Erstreckung kann sichergesellt werden, dass ein unnötiges Verblocken der offenen Bereiche im Rahmen der Fertigungstoleranzen zuverlässig vermieden werden kann, wenn das Windelverschlussband um einen Windelrand herumgefaltet wird.

Es hat sich herausgestellt, dass die Verschlusskraft des zwei-komponentigen mechanischen Verschlusssystems erhöht werden kann, wenn die erste Komponente des zwei-komponentigen mechanischen Verschlusssystems wenigstens zwei voneinander beabstandete Komponentenstreifen umfasst. Insbesondere drei und vier nebeneinander angeordneten Komponentenstreifen erweisen sich ebenfalls als vorteilhaft, wobei weit mehr als diese Anzahl an Komponentenstreifen lediglich bei sehr langen und großen Windelverschlussbändern praktikabel erscheint.

Insbesondere kann zwischen den beiden oder auch den mehreren voneinander beabstandeten Komponentenstreifen die Klebstoffschicht wenigstens einen offenen Bereich aufweisen, der dann ebenfalls eine entsprechende Niederhaltefunktion ausüben kann. Auf diese Weise kann über die Fläche des Fastening-Bereichs eine gute Niederhaltefunktion bzw. eine sichere Verbindung zu dem textilen Oberflächenbereich gewährleistet werden.

Der offene Bereich zwischen den Komponentenstreifen sollte vorzugsweise zumindest 0,3 mm, insbesondere zumindest 0,4 mm, breit sein, damit die Klebstoffschicht in ausreichendem Maße niederhaltend wirksam werden kann.

Andererseits erscheint es vorteilhaft, wenn der offene Bereich zwischen den Komponentenstreifen nicht mehr als 2,5 mm, insbesondere nicht mehr als 2,3 mm, breit ist, sodass die klebende Niederhaltefunktion das Verhalten des Fastening-Bereichs bzw. des mechanischen Windelverschlussbands nicht zu sehr dominiert.

Alternativ bzw. kumulativ hierzu kann ein einzelner offener Bereich zwischen den Komponentenstreifen zumindest 8%, vorzugsweise zumindest 10%, der Breite eines der Komponentenstreifen breit sein, sodass sichergestellt ist, dass dieser offene Bereich zwischen den Komponentenstreifen zu der Niederhaltefunktion in ausreichendem Maße beitragen kann.

Dementsprechend kann es kumulativ bzw. alternativ hierzu von Vorteil sein, wenn ein einzelner offener Bereich nicht mehr als 100 %, vorzugsweise nicht mehr als 90 %, der Breite eines der Komponentenstreifen breit ist, sodass dieser offene Bereich die an sich gewünschten mechanischen Verschlusseigenschaften nicht zu sehr dominiert, wobei - auch in diesem Zusammenhang - zu berücksichtigen ist, dass möglicherweise zu breite offene Bereiche die Gefahr von Faserausrissen durch die zugehörige Klebstoffschicht in unerwünschtem Maße erhöhen können.

Es versteht sich, dass die Merkmale der vorstehend bzw. in den Ansprüchen beschriebenen Lösungen gegebenenfalls auch kombiniert werden können, um die Vorteile entsprechend kumuliert umsetzen zu können.

Weitere Vorteile, Ziele und Eigenschaften vorliegender Erfindung werden anhand nachfolgender Beschreibung von Ausführungsbeispielen erläutert, die insbesondere auch in anliegender Zeichnung dargestellt sind. In der Zeichnung zeigen:
- Figur 1: schematisch ein Windelverschlussband in einer exemplarischen Seitenansicht;
- Figur 2: das Windelverschlussband nach Fig. 1 in einem Bereitstellungszustand an einer Windel in einem der Blickrichtung nach Fig. 2 entsprechenden, schematischen Schnitt durch die Windel;
- Figur 3: die Anordnung nach Fig. 2, bei welcher das Windelverschlussband in einem Arbeitszustand verbracht ist und die Windel verschließt, in einer ähnlichen Darstellung wie Fig. 2;
- Figur 4: die Anordnung nach Fign. 2 und 3 zu Beginn eines 2*90°-Tests, in einer ähnlichen Darstellung wie Fign. 2 und 3;
- Figur 5: die Anordnung nach Fign. 2 bis 4 während des 2*90°-Tests, in einer ähnlichen Darstellung wie Fign. 2 bis 4;
- Figur 6A: erste exemplarische Messergebnisse für den 2*90°-Test;
- Figur 6B: zweite exemplarische Messergebnisse für den 2*90°-Test;
- Figur 6C: dritte exemplarische Messergebnisse für den 2*90°-Test;

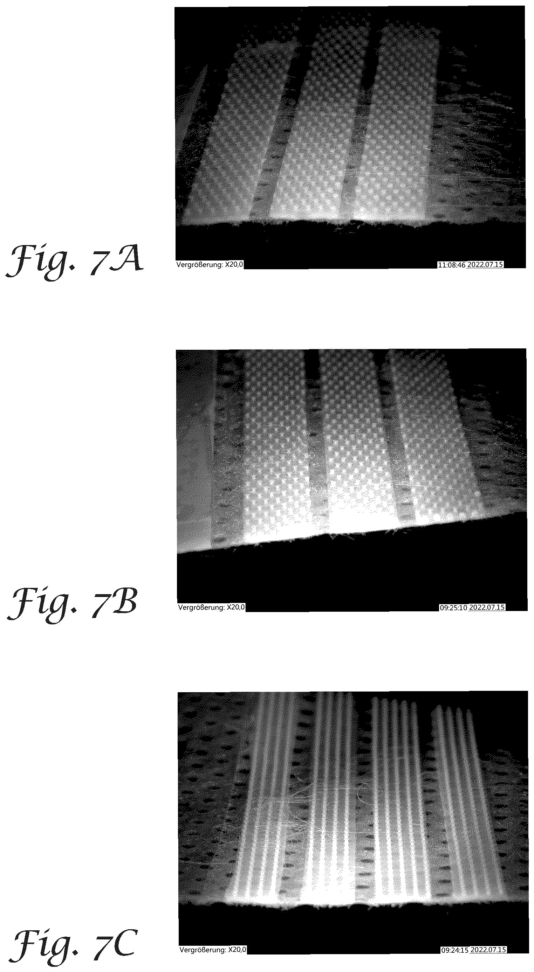

- Figur 7A: eine fotographische Darstellung des Fastening-Bereichs eines der Teststreifen zur Durchführung des in Fig. 6A dargestellten 2*90°-Tests;
- Figur 7B: eine fotographische Darstellung des Fastening-Bereichs eines der Teststreifen zur Durchführung des in Fig. 6B dargestellten 2*90°-Tests
- Figur 7C: eine fotographische Darstellung des Fastening-Bereichs eines der Teststreifen zur Durchführung des in Fig. 6C dargestellten 2*90°-Tests
- Figur 8A: eine fotographische Darstellung eines ersten Windelrands mit einem textilen Oberflächenbereich und eines zugehörigen Windelverschlussbands beim Öffnen der Verbindung des Fastening-Bereichs zu dem textilen Oberflächenbereich;
- Figur 8B: eine fotographische Darstellung eines zweiten Windelrands mit einem textilen Oberflächenbereich und eines zugehörigen Windelverschlussbands beim Öffnen der Verbindung des Fastening-Bereichs zu dem textilen Oberflächenbereich;
- Figur 9A: schematisch eine Anordnung zur Durchführung einer loop-tack-Prüfung;
- Figur 9B: exemplarische Messergebnisse für die loop-tack-Prüfung;
- Figur 10A: schematisch eine Anordnung zur Durchführung einer peel-adhesion-Prüfung;
- Figur 10B: exemplarische Messergebnisse für die peel-adhesion-Prüfung;
- Figur 11: ein graphischer Vergleich unterschiedlicher Klebstoffe, die als Klebstoffschicht zur Anwendung kommen, ergänzt um eine Einordnung des vorliegend vorgestellten Klebstoffs;
- Figur 12: ein Halbzeugband zur Bereitstellung eines Windelverschlussbands in Form einer Rolle; und
- Figur 13: ein Halbzeugband zur Bereitstellung eines Windelverschlussbands in Form einer Spule.

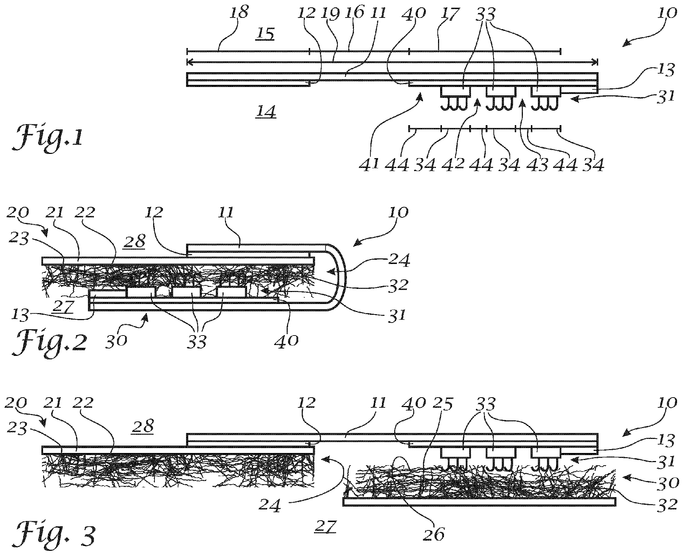

Das in Figur 1 dargestellte Windelverschlussband 10 umfasst ein Trägerband 11, welches sich, ebenso wie das Windelverschlussband 10, entlang einer Länge 19 erstreckt.

Das Windelverschlussband 10 bzw. das Trägerband 11 weisen einen Fastening-Bereich 17 und einen Permanent-Bereich 18 auf, welche entlang der Länge 19 des Trägerbands 11 bzw. des Windelverschlussbands 10 versetzt zueinander angeordnet sind.

Bei vorliegendem Ausführungsbeispiel sind der Fastening-Bereich 17 und der Permanent-Bereich 18 durch einen Abstand 16 entlang der Länge 19 voneinander beabstandet.

In abweichenden Ausführungsformen ist ein derartiger Abstand nicht zwingend erforderlich, wobei sich herausgestellt hat, dass durch einem Abstand zum einen Spiel in Bezug auf Fertigungstoleranzen ermöglicht und beispielsweise ein etwaiges Verblocken des Windelverschlussbands vermieden werden kann, wenn dieses, wie in Figur 2 exemplarisch dargestellt, einen Windelrand 24 einer Windel 20 umgreifend an der Windel 20 angebracht wird. Zum anderen kann durch einen derartigen Abstand 16 die Handhabe des Windelverschlussbands 10 durch einen Nutzer erleichtert werden.

Das Windelverschlussband 10 weist an einer Innenseite 14 in dem Permanent-Bereich 18 eine Permanentklebstoffschicht 12 auf, mittels welcher das Windelverschlussband 10 permanent an der Windel zu befestigen ist.

Die Permanentklebstoffschicht 12 ist hierbei derart ausgestaltet, dass das Windelverschlussband 10 bei bestimmungsgemäßem Gebrauch in dem Permanent-Bereich 18 nicht zerstörungsfrei von der Windel 20 zu entfernen ist. Es ist jedoch denkbar, dass beispielsweise durch einen geeigneten Einsatz von Lösungsmitteln oder durch gezielte mechanische Eingriffe ein Ablösen gelingen kann.

Da der Fastening-Bereich 17 derart ausgebildet ist, dass er eine öffenbare Verbindung zu bestimmten Oberflächenbereichen 22, 26 der Windel 20 eingehen kann, erscheint es jedoch auch nicht erforderlich, dass Nutzer sich bemühen, dass Windelverschlussband 10 im Permanent-Bereich 18 von der Windel zu lösen. Insofern kann der Klebstoff, welcher als Permanentklebstoffschicht 12 für den Permanent-Bereich 18 genutzt wird, auch durchaus ein zerstörungsfreies Öffnen, solange dieses unter einem gewissen Aufwand erfolgen muss, der eigentlich permanenten Verbindung an der Windel 20 ermöglichen.

Das Windelverschlussband 10 sowie das Trägerband 11 weisen darüber hinaus eine Außenseite 15 auf, welche der Innenseite gegenüberliegend angeordnet ist.

In dem Fastening-Bereich 17 dienen eine erste Komponente 31 eines zwei-komponentigen mechanischen Verschlusssystems 30 sowie offene Bereiche 41, 42, 43 einer Klebstoffschicht 40 dem Bereitstellen einer öffen- und wiederverschließbaren Verbindung des Fastening-Bereichs 17 und somit auch des Windelverschlussbands 10 zu Oberflächenbereichen 22, 26 der Windel 20.

In konkreter Umsetzung ist bei diesem Ausführungsbeispiel auf der Innenseite 14 des Fastening-Bereichs 17 eine durchgängige Klebstoffschicht 40 vorgesehen, mittels welcher die erste Komponente 31 des zwei-komponentigen mechanischen Verschlusssystems 30 an dem Trägerband 11 angebracht ist.

Bei vorliegendem Ausführungsbeispiel ist die erste Komponente 31 des zwei-komponentigen mechanischen Verschlusssystems 30 in Form von Komponentenstreifen 33, welche eine parallel zur Länge 19 des Windelverschlussbands 10 ausgerichtete Breite 34 aufweisen, an dem Trägerband 11 angebracht.

Unabhängig von etwaigen weiteren Maßnahmen ermöglicht die Ausgestaltung der ersten Komponente 31 in Form von Komponentenstreifen 33 eine höhere Flexibilität der ersten Komponente 31 des zwei-komponentigen mechanischen Verschlusssystems 30, was sich in der Praxis als vorteilhaft hinsichtlich der mechanischen Verschlusskräfte zu einer zweiten Komponente 32 des zwei-komponentigen mechanischen Verschlusssystems 30 herausgestellt hat. Es versteht sich, dass eine entsprechende Flexibilisierung auch durch andere Ausgestaltungen der ersten Komponente 31 des zwei-komponentigen mechanischen Verschlusssystems 30 realisiert werden kann, indem beispielsweise separate Flecken oder Inseln bzw. Materialstreifen mit Ausstanzungen statt der Komponentenstreifen 33 genutzt werden. Bei dem vorliegenden Ausführungsbeispiel sind die Komponentenstreifen 33 geradlinig ausgebildet. Je nach konkreter Umsetzung können die Komponentenstreifen 33 in abweichenden Ausführungsform jedoch auch von einer gradlinigen Struktur abweichende Formen, wie beispielsweise eine Wellenstruktur aufweisen.

Die Komponentenstreifen 33 sind voneinander beabstandet, sodass zwischen diesen offene Bereiche 42, 43 mit einer Breite 44 verbleiben. Es versteht sich, dass in abweichenden Ausführungsformen entsprechende Abstände nicht vorgesehen sein müssen, solange ansonsten offene Bereiche für eine klebend wirksame Niederhaltefunktion verbleiben.

Ergänzend bzw. alternativ ist die Klebstoffschicht 40 in Richtung des Permanent-Bereichs 18 um eine Breite 44 verlängert, sodass ein weiterer offener Bereich 41 zur Ausübung einer Niederhaltefunktion bereitgestellt ist.

In abweichenden Ausführungsformen kann die Klebstoffschicht 40 beispielsweise in Richtung des Permanent-Bereichs 18 weiterverlängert werden, um auf diese Weise beispielsweise den Fastening-Bereich 17 zu vergrößern und auch die klebende Niederhaltefunktion zu stärken. Da es sich jedoch vorliegend um ein Windelverschlussband 10 handelt, welches mechanisch wirken soll, sollte die Größe der offenen Bereiche 41, 42, 43 derart gewählt werden, dass der klebende Charakter des Windelverschlussbands 10 nicht überhandnimmt. Gegebenenfalls kann die Klebstoffschicht 40 bis zu dem Permanent-Bereich 18 reichen, was jedoch gegebenenfalls zu Fertigungsproblemen führen kann, da bei einem Umfalten um einen Windelrand dann die Gefahr einer Verblockung besteht.

Je nach konkreter Wahl des Klebstoffs für die Klebstoffschicht 40 kann die Klebstoffschicht auch bis in den Permanent-Bereich 18 reicht, sodass diese auch die Permanentklebstoffschicht 12 darstellen kann. Selbst bei einer nicht durchgängigen Klebstoffschicht 40 ist es denkbar, für die Klebstoffschicht 40 und die Permanentklebstoffschicht 12 einen identischen Klebstoff zu verwenden. Dieses kann sich insbesondere dann als durchführbar und zweckmäßig erweisen, wenn der Permanent-Bereich 18 gegen Oberflächen der Windel 20 geklebt werden soll, welche von den textilen Oberflächen Bereichen 22, 26 mit denen der Fastening-Bereich 17 die wiederöffenbare Verbindung eingehen soll, abweichen.

Wird eine bis zu oder sogar in den Permanent-Bereich 18 reichende Klebstoffschicht 40 genutzt, so kann es von Vorteil sein, diese Klebstoffschicht zwischen einem gewünschten Fastening-Bereich 17 und dem gewünschten Permanent-Bereich 18 durch eine Abdeckung zu kaschieren oder sonstwie zu deaktivieren. Auch auf diese Weise kann ein Abstand 16 zwischen dem Fastening-Bereich 17 und dem Permanent-Bereich 18 baulich einfach bereitgestellt werden.

Auf der von dem Permanent-Bereich 18 wegweisenden Seite des Fastening-Bereichs 17 ist darüber hinaus für das Windelverschlussband 10 ein Fingerlift 13 vorgesehen. Ein derartiger Fingerlift 13 kann auf vielfältige Weise bereitgestellt werden. Beispielsweise kann einfach ein offener Bereich des Trägerbands 11 als Fingerlift 13 dienen. Es hat sich jedoch als vorteilhaft erwiesen, den Fingerlift 13 durch eine ergänzende Laminierung bereitzustellen, da dann der Fingerlift 13 einfach zu ergreifen ist. Gegebenenfalls kann für ein Bereitstellen des Fingerlifts 13 das Trägerband 11 in sich umgefaltet werden, um auf diese Weise eine dickere Schichtstruktur bereitzustellen, welche einfacher ergriffen werden kann. Darüber hinaus ermöglicht eine Kaschierung für den Fingerlift 13, diesen gegebenenfalls farbig oder auf sonstige Weise haptisch besonders auszugestalten, sodass dieser einfacher als Fingerlift 13 erkennbar ist, wenn ein Nutzer das Windelverschlussband 10 nutzen möchte. Je nach konkreter Umsetzung kann gegebenenfalls auf einen Fingerlift 13 auch zur Gänze verzichtet werden, wenn beispielsweise die erste Komponente 31 zwei-komponentigen mechanischen Verschlusssystems 30 ein ausreichendes Ergreifen des Windelverschlussbands 10 in der Nähe des Fastening-Bereichs 17 ermöglicht.

Das Trägerband 11 kann, wie an sich hinlänglich am Markt und aus dem Stand der Technik bekannt, als Folie, als verfestigtes Nonwoven, aus textilem Material, aus Papier, aus Kunststoff sowie aus Laminaten hieraus bereitgestellt werden. Bei vorliegendem Ausführungsbeispiel ist das Trägerband durch ein verfestigtes Nonwoven bereitgestellt. Es versteht sich, dass auch andere Materialien genutzt werden können, solange an deren Innenseite 14 entsprechende Fastening-Bereiche 17 und Permanent-Bereiche 18 bereitgestellt werden können sowie das dann gebildete Windelverschlussband 10 ausreichend beweglich und den für die Verbindung bzw. das Schließen notwendigen Kräften in ausreichendem Maße begegnen kann.

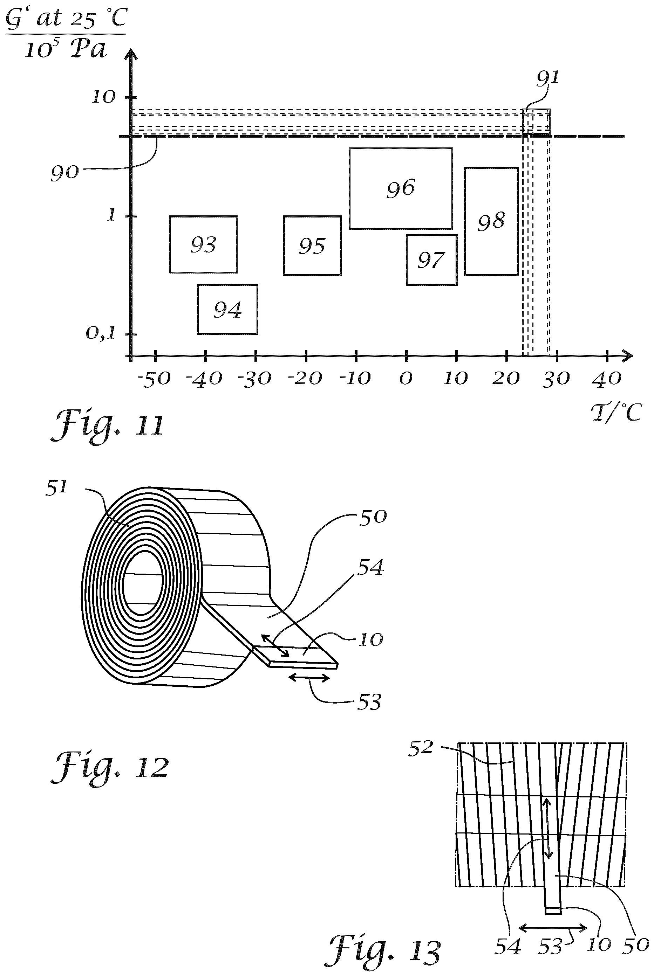

In der Fertigung hat es sich bewährt, derartige Windelverschlussbänder 10 aus Halbzeugbändern 50 bereitzustellen, wie diese beispielhaft in Figuren 12 und 13 dargestellt sind.

Derartige Halbzeugbänder 50 können entlang einer Maschinenlaufrichtung 54 mit verhältnismäßig hohen Laufgeschwindigkeiten laminiert werden, wenn anschließend die Halbzeugbänder 50 in einer senkrecht zur Maschinenlaufrichtung 54 liegenden Querrichtung 53 zu den Windelverschlussbändern 10 durchtrennt werden. Letzteres kann insbesondere durch einen geeigneten Schneidevorgang erfolgen.

Je nach konkretem Fertigungsprozess können hierbei die Halbzeugbänder 50 gegebenenfalls auch mehrnutzig bereitgesellt werden, was bedeutet, dass in Querrichtung des Halbzeugsbands 50 eine Aufbau bereitgestellt wird, aus dem durch Trennungen in Maschinenlaufrichtung 54 mehrere Windelverschlussbänder 10 gewonnen werden können. Hierbei ist es letztlich unerheblich, ob zunächst die Trennung des Halbzeugbands 50 in Querrichtung 53 erfolgt, um anschließend die Trennung in Maschinenlaufrichtung 54 vorzunehmen, oder umgekehrt, solange am Ende die Windelverschlussbänder 10 in gewünschter Form bereitstehen und etwaige Transport- oder Lagervorgänge kostengünstig und effektiv durchgeführt werden können.

Letztlich kann ein derartiges Halbzeugband 50 in jeder Form bereitgestellt werden, in der eine maschinelle Verarbeitung möglich ist. Insbesondere ist es denkbar, dass derartige Halbzeugbänder 50 in einem losen Haufen gelagert und transportiert werden. Vorteilhaft ist es jedoch, wenn derartige Halbzeugbänder 50 in Form von Rollen 51, wie in Figur 12 exemplarisch dargestellt, oder Spulen 52, wie in Figuren 13 exemplarisch dargestellt, gewickelt werden, was gute Transport- und Lagermöglichkeiten eröffnen.

Wie bereits vorstehend dargelegt, soll das Windelverschlussband 10 zum einen permanent mit dem Permanent-Bereich 18 an einer Windel 20 befestigt werden und zum anderen, einen Windelrand 24 umgreifend, mit seinem Fastening-Bereich 17 lösbar mit der Windel 20 verbunden werden, wie dieses exemplarisch in Figur 2 angedeutet ist.

Bei vorliegendem Ausführungsbeispiel ist der Windelrand 24 an einem Windelohr der Windel 20 vorgesehen, welches zur Windelinnenseite 27 hin textil ausgebildet ist, während dieses zur Windelaußenseite 28 hin eine folienartige Schicht umfasst.

Auf diese Weise wird ein textiler Oberflächenbereich 22 an der Windelinnenseite 27 bereitgestellt, welche als zweite Komponente 32 des zwei-komponentigen mechanischen Verschlusssystems 30 dienen kann, sodass die erste Komponente zwei-komponentigen mechanischen Verschlusssystems 30 mit dieser zweiten Komponente 32 eine öffenbare mechanische Verbindung eingehen kann, wie dieses exemplarisch in Figur 2 dargestellt ist.

Je nach konkreter Ausgestaltung kann der textile Oberflächenbereich 22 auch an dem Windelkörper der Windel 20 und nicht an einem Windelohr der Windel 20 ausgebildet sein. Ebenso kann der textile Oberflächenbereich 22, je nach konkreter Ausgestaltung, durch ein Nonwoven, ein Gewebe, ein Gewirke oder durch eine sonstige Struktur mit textilem Charakter bereitgestellt werden, solange eine ausreichende Wechselwirkung mit der ersten Komponente 31 des zwei-komponentigen mechanischen Verschlusssystems 30 möglich ist und den übrigen Anforderungen an die Windelinnenseite 27 einer Windel 20, beispielsweise durch ein ausreichend angenehmes haptisches Gefühl, in ausreichendem Maße Rechnung getragen ist.

Statt der folienartigen Schicht an der Windelaußenseite 28 kann gegebenenfalls auch das Material des textilen Oberflächenbereichs 22 genutzt werden, wenn dieses ausreichend eigenstabil bzw. verfestigt ist. Ebenso ist es denkbar, dass textiles Material auch an der Windelaußenseite 28 auf die folienartige Schicht aufkaschiert ist, insbesondere wenn die Windel 20 auch an der Außenseite 15 entsprechende haptische Eigenschaften aufweisen soll.

Bei vorliegendem Ausführungsbeispiel dient die folienartige Schicht als Trägerschicht 21 für den textilen Oberflächenbereich 22, kann jedoch auch abweichend hiervon ausgestaltet sein.

Das vorliegende zwei-komponentige mechanischen Verschlusssystems 30 weist als erste Komponente 31 ein auch als Klettmaterial bezeichnetes Hakenmaterial auf, welche in Fasern 23 textiler Oberflächenbereiche 22, 26 der Windel 20 eingreifen kann, sodass diese textile Oberflächenbereiche 22, 26 als Schlaufenmaterial bezeichnet werden können, sodass es sich vorliegend bei dem zwei-komponentigen mechanischen Verschlusssystems 30 um eine Haken-Schlaufen-Verbindung handelt. Es versteht sich, dass in abweichenden Ausführungsformen auch andere zwei-komponentige mechanische Verschlusssysteme 30, wie beispielsweise Haken-Ösen-Verbindungen oder ähnliches zur Anwendung kommen können, solange zwei aufeinander abgestimmte Komponenten gemeinsam das Verschlusssystem bilden. Hierbei brauchen die textilen Oberflächenbereiche 22, 26 nicht zwingend einzeln ausgebildete und stabilisierte Schlaufen aufweisen, wie dieses bei der Verwendung von Geweben und Gewirken hinlänglich bekannt ist. Vielmehr reicht ein ausreichend verfestigter Faserverbund bereits aus, um durch einzelne Fasern schlaufenartige Bereiche bereitzustellen, in welche die Haken eindringen können.

Dem gegenüber ist eine Klebstoffschicht eher als ein-komponentiges Verschlusssystem anzusehen, da diese Schicht alleine dafür verantwortlich ist, zwei Baugruppen miteinander in der gewünschten Weise zu verbinden.

In dem in Figur 2 dargestellten Bereitstellungszustand umgreift das Windelverschlussband 10 den Windelrand und ist an der Windelaußenseite 28 permanent an der Windel 20 befestigt. An der Windelinnenseite 27 liegt eine öffenbare Verbindung einerseits durch das zwei-komponentigen mechanischen Verschlusssystems 30 und andererseits durch die offenen Bereiche 41, 42, 43 der Klebstoffschicht 40 vor.

In diesem Bereitstellungszustand liegt das Windelverschlussband verhältnismäßig eng und relativ kontrolliert an der Windel 20 an. Lediglich ein kurzer Bereich, nämlich der Bereich des Fingerlifts 13, ist vorzugsweise zu unkontrollierten Bewegungen fähig. Auf diese Weise kann gewährleistet werden, dass das Windelverschlussband 10 in dieser Bereitstellungsposition während des Produktions- und Verpackungsprozesses betriebssicher verbleibt, bis ein Nutzer die Windel 20 aus ihrer Verpackungseinheit holt und anwenden möchte.

Im Anwendungsfall ist es dann an beispielsweise an dem Nutzer, das Windelverschlussband 10 von dem in Figur 2 dargestellten Bereitstellungszustand in einen Arbeitszustand zu verbringen, wie dieser exemplarisch in Figur 3 dargestellt ist.

Hierfür öffnet der Nutzer die mechanische Verbindung des zwei-komponentigen mechanischen Verschlusssystems 30 und die Verbindung der offenen Bereiche 41, 42, 43 von dem textilen Oberflächenbereich 22 und streckt das Windelverschlussband 10 in gewisser Weise. Anschließend ist es möglich, den Fastening-Bereich 17 an anderen textilen Oberflächenbereichen 26 der Windel 20 anzusetzen und dort zumindest eine mechanische Verbindung zu schaffen. Je nach konkreter Ausführungsform können die offenen Bereiche 41, 42, 43 noch ausreichend taktil sein, um auch in Bezug auf die textilen Oberflächenbereiche 26 eine Niederhaltefunktion auszuüben.

Je nach konkretem Anwendungsfall kann der Nutzer die in Figur 3 exemplarisch dargestellte Verbindung wieder öffnen, um beispielsweise den Sitz der Windel 20 zu kontrollieren oder zu ändern, um die Windel 20 auf einen etwaigen Inhalt zu prüfen oder um die Windel 20 gegebenenfalls zu entfernen und zu entsorgen. Je nach konkreter Ausgestaltung der Windelaußenseite 28 kann das Windelverschlussband 10 auch zum Verschließen der 20 in einer Entsorgungsposition dienen.

Damit der Fastening-Bereich 17 an der Windelaußenseite 28 in geeigneter Weise angesetzt werden kann, ist an der Windelaußenseite 28 wenigstens eine Landezone 25 ausgebildet, in welcher der textile Oberflächenbereich 26 zu finden ist. Eine derartige Landezone 25 kann beispielsweise als Frontaltape separat an der Windelaußenseite 28 angesetzt sein. Ebenso ist es denkbar, dass derartige Landezonen noch an weiteren Positionen vorgesehen sind, um ein Befestigen des jeweiligen Fastening-Bereichs 17 in anderen Positionen, beispielsweise in einer Entsorgungsposition, zu ermöglichen. Gegebenenfalls kann sogar auf der Außenseite 15 des Windelverschlussbands 10 eine Landezone 25 der Windel 20 vorgesehen sein. Ebenso ist es möglich, die gesamte Außenschicht der Windel 20 oder zumindest den überwiegenden Teil der Außenschicht der Windel 20 als textilen Oberflächenbereich 26 auszubilden, sodass sehr großflächig eine Landezone 25 bereitgestellt ist.

Es hat sich herausgestellt, dass ein Ausreißen von Fasern 23 der textilen Oberflächenbereiche 22, 26 beim Abheben des Fastening-Bereichs 17 von den textilen Oberflächenbereichen 22, 26 dazu führt, dass diese Fasern ein Wiederverschließen des Fastening-Bereichs 17 verhindern. Hierbei hat sich des Weiteren herausgestellt, dass insbesondere die freien Bereiche 41, 42, 43 der Klebstoffschicht 40 stark zu einer entsprechenden Kontamination des Fastening-Bereichs 17 beitragen können. Dementsprechend ist es vorteilhaft, wenn die Klebstoffschicht 40 derart ausgebildet ist, dass sie einerseits einen sicheren Sitz der ersten Komponente 31 des zwei-komponentigen mechanischen Verschlusssystems 30 an dem Trägerband 11 sowie eine ausreichende klebende Niederhaltefunktion zumindest in dem Bereitstellungszustand gewährleisten und andererseits jedoch beim Übergang in den in Figur 3 dargestellten Arbeitszustand möglichst wenig, wenn gar keine Fasern durch die Klebstoffschicht 40 herausreissen.

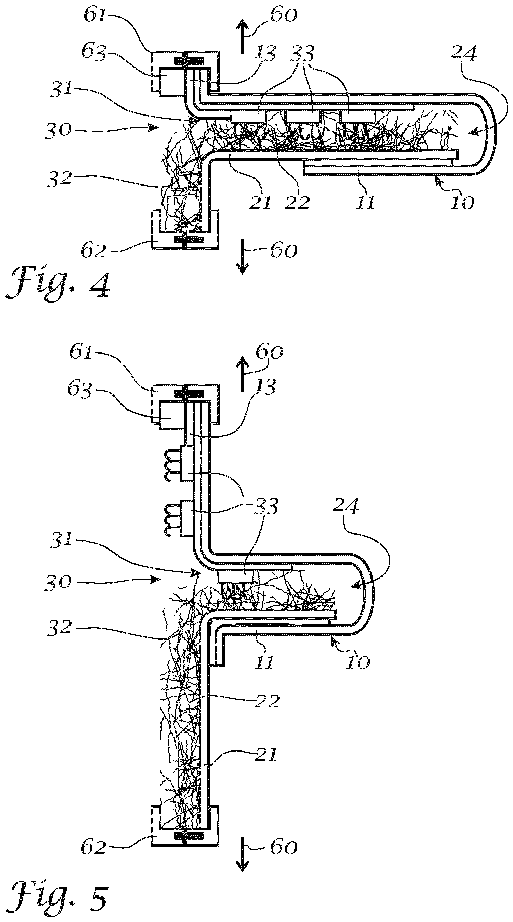

Durch eine peel-Mindestkraftmessung, wie sie in Figuren 4 und 5 exemplarisch dargestellt ist, kann ein Klebstoff beispielsweise im Hinblick auf seine ausreichende Niederhaltefunktion und auf seine Neigung bzw. fehlende Neigung, Fasern 23 aus den textilen Oberflächenbereichen 22 oder 26 auszureißen, geprüft werden.

Vorzugsweise erfolgt die Prüfung ausgehend von dem Bereitstellungszustand (s. Figur 2) und somit in Bezug auf den textilen Oberflächenbereich 22.

Für die Prüfung kann beispielsweise eine Verpackungseinheit von Windeln 20 zunächst über 24 Stunden auf 23 ± 2 °C und 50 ± 5% relative Luftfeuchtigkeit klimatisiert werden. Anschließend werden exemplarisch Windeln 20 aus der Verpackungseinheit entnommen und die textilen Oberflächenbereiche 22 gemeinsam mit den sie umgreifenden Windelverschlussbändern 10 ausgeschnitten.

Für die peel-Mindestkraftmessung kann beispielsweise ein Zugfestigkeitsmessgerät genutzt werden, welches zwei Klemmbacken 61, 62 aufweist.

In die untere Klemmbacke 62 kann dann beispielsweise der textile Oberflächenbereich 22 eingeklemmt werden, während in die obere Klemmbacke 61 des Zugprüfgeräts der Fingerlift, gegebenenfalls gemeinsam mit einem Verstärkungsband 63 eingeklemmt wird.

Anschließend werden die Klemmbacken 61, 62 durch das Zugprüfgerät in Bewegungsrichtung 60 auseinander bewegt, sodass ein 2*90°-Test durchgeführt werden kann, bei welchen der Kraftverlauf aufgezeichnet, woraus Minimal- bzw. Maximalkräfte ermittelt werden können.

Vorzugsweise werden mehrere Prüflinge vermessen, sodass aus den Messergebnissen Mittelwerte und Messgenauigkeitsgrenzen ermittelt werden können.

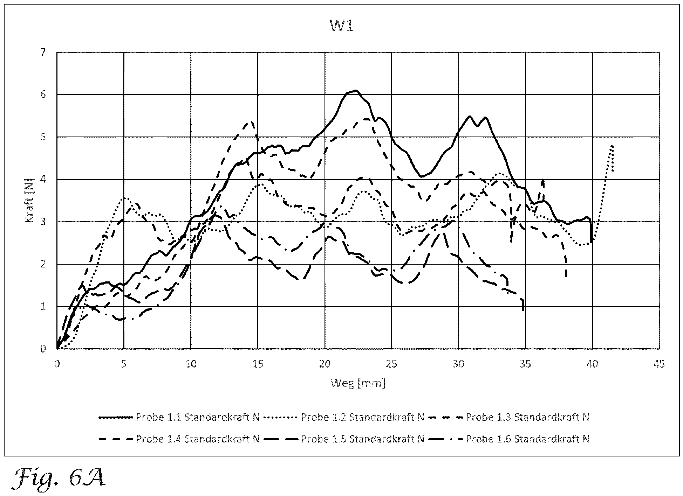

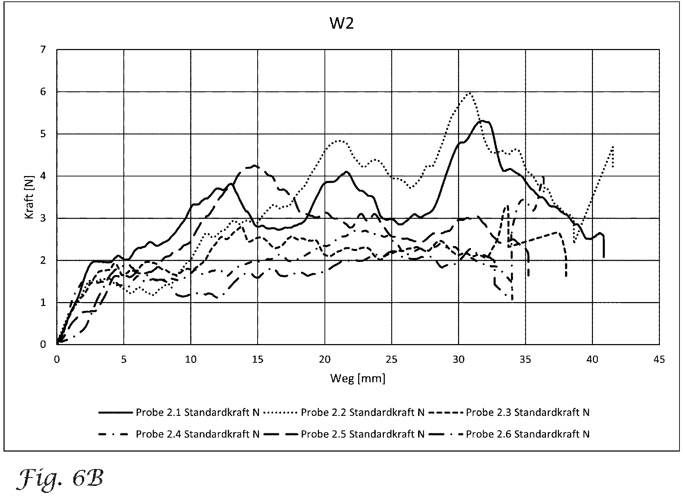

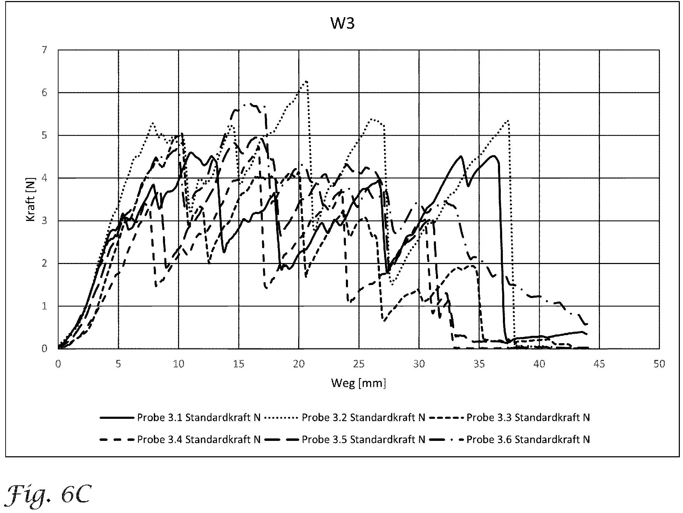

Entsprechende Messergebnisse zeigen die Figuren 6A bis 6C, wobei diese Messergebnisse entnehmbar ist, dass die Klebstoffschicht einem Öffnen der Verbindung eine peel-Mindestkraft von wenigstens 0,6 N/(25 mm), im Konkreten sogar von wenigstens 1 N/(25 mm) entgegensetzt, wenn Artefakte in Randbereichen ausgeklammert werden.

Eine Sichtung der Fastening-Bereiche exemplarischer Prüflinge zeigt, dass äußerst wenige Fasern bei diesem Test ausgerissen wurden. Die Zahl der Fasern liegt deutlich unter 15 pro 0,125 cm².

Auch lässt sich diesem Messergebnissen entnehmen, dass die Peel-Mindestkraft um wenigsten 0,7 N/(25 mm) geringer ist, als die Maximalkraft, welche das zwei-komponentige mechanische Verschlusssystem 30 dem Öffnen der Verbindung entgegensetzt.

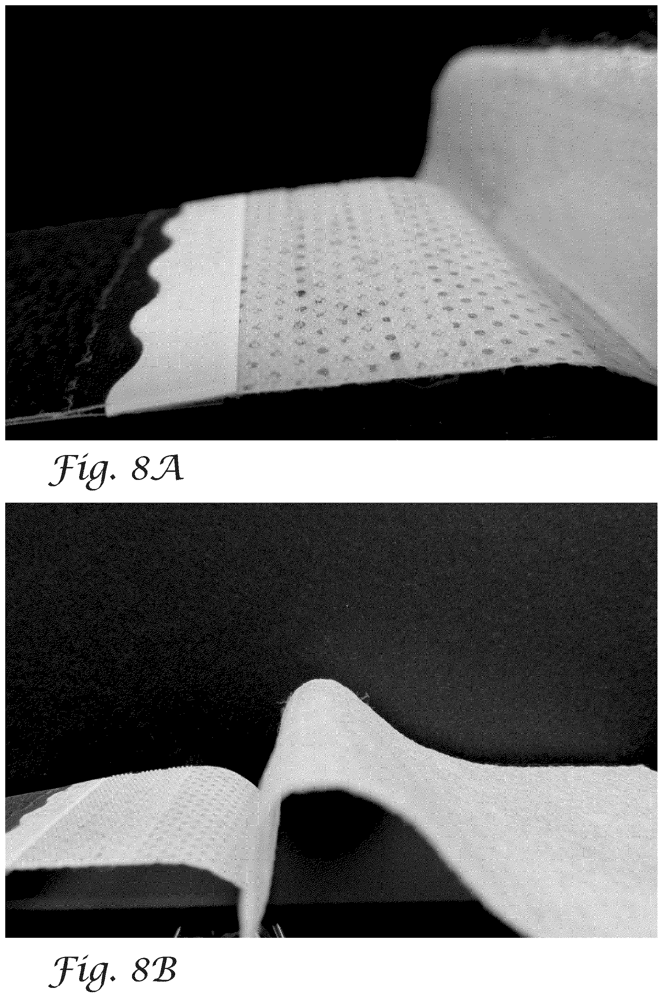

Bei den Darstellungen der Figuren 8A und 8B wurden die Fastening-Bereiche 17 aus dem Bereitstellungszustand (s. Figur 2) händisch geöffnet. Bei diesem Vorgang stellt sich heraus, dass keine Fasern sichtbar aus dem textilen Oberflächenbereich 22 ausgerissen wurden.

Zur Bestimmung der Sofortklebekraft des für die Klebstoffschicht 40 vorgesehenen Klebstoffs wird ein dem Trägerband 11 entsprechendes Materialband großflächig mit diesem Klebstoff 40 beschichtet. Faktisch kann dieses beispielsweise dadurch bereitgestellt werden, dass währen des Laminierens des Halbzeugbands ein Auflaminieren der Komponentenstreifen 33 verhindert wird, sodass entlang der Maschinenlaufrichtung ein entsprechend langer Streifen der Klebstoffschicht 40 zur Verfügung steht.

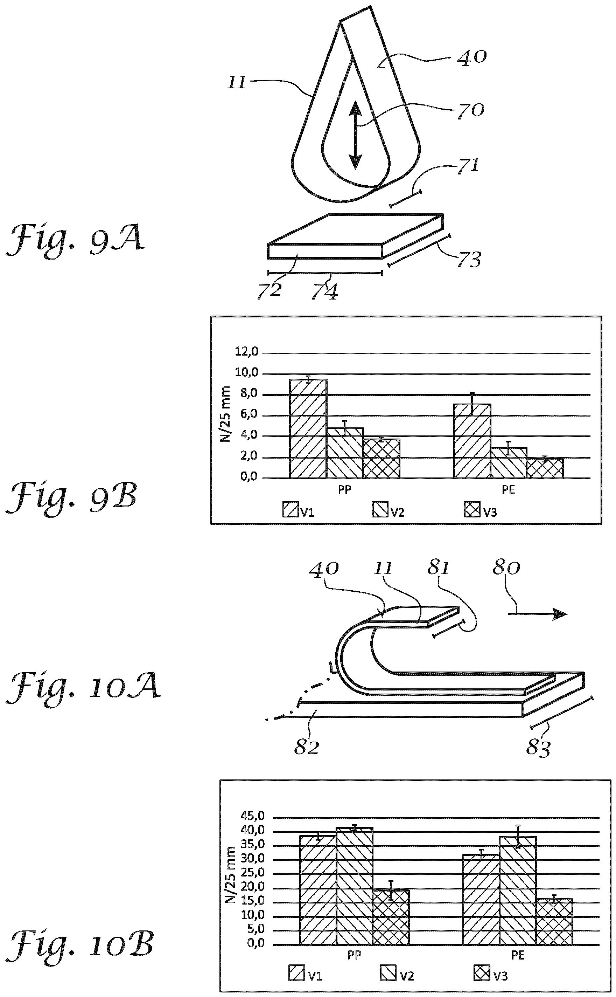

Das entsprechende Material wird dann für mindestens vier Stunden bei 23 ±2 °C und 50 ± 5% relativer Luftfeuchtigkeit klimatisiert und anschließend, wie in Figur 9A exemplarisch dargestellt, zu einer Schlaufe geformt, bei welcher die Klebstoffschicht 40 außen liegt. Der obere Teil der Schlaufe wird in die obere Klemmbacke eines Zugprüfgeräts eingespannt, wobei sich empfiehlt, den Klebstoff im Bereich der Klemmbacke zu kaschieren, um Verschmutzungen zu vermeiden.

In die untere Klemmbacke des Zugprüfgeräts wird eine Prüfplatte aus Polyethylen (PE) oder Polypropylen (PP) eingespannt, wobei die Prüfplatte bei vorliegender Messung eine Länge 74 von 70 mm aufweist, was letztlich den Trennweg bzw. den Prüfweg darstellt. Die Breite 73 der Prüfplatte 72 ist größer 30 mm gewählt und spielt letztlich keine Rolle, solange diese Breite 73 größer ist als die Breite 71 des für die loop-track-Prüfung genutzten Trägerbands 11 ist.

Nach den entsprechenden Vorbereitungen wird das Zugprüfgerät in Bewegungsrichtung 70 bewegt, sodass die Klemmbacken aufeinander zu fahren, bis die Schlaufe des Trägerbands 11 die Länge 74 der Prüfplatte 72 vollständig überdeckt. Dann wird sofort die Bewegungsrichtung 70 umgekehrt.

Entsprechende Messergebnisse zeigt die Figur 9B, wobei insbesondere der jeweils rechts dargestellte Klebstoff keine bzw. sehr niedrige Faserausrisse gewährleistet.

Für die Quantifizierung der Klebkraft kann eine peel-adhesion-Prüfung durchgeführt werden, bei welcher ein Trägerband 11, wie es bereits für die Loop-Tack-Prüfung beschrieben wurde, mit einer FINAT-Standard-Anpressrolle mit einer Geschwindigkeit von ungefähr 10 mm pro Sekunde zweimal in beide Richtung auf eine Prüfplatte 82 geklebt und überrollt wird, um einen guten Kontakt zwischen dem Trägerband 11 und der Prüfplatte 82 zu gewährleisten.

Auch bei dieser Messung ist die Breite 83 der Prüfplatte 82 größer gewählt als die Breite 81 des für die peel-adhesion-Prüfung genutzten Trägerbands 11.

Nach einer Wartezeit von 20 Minuten bzw. 24 Stunden folgt dann die entsprechende Prüfung, wobei die Testplatte so in dem Zugprüfgerät befestigt wird, dass das Trägerband 11 einen Abzugwinkel von 180° erhält. Dann werden die Klemmen des Zugprüfgeräts mit 300 mm pro Minute auseinander gefahren. Während der Prüfung wird die Kraft mindestens fünfmal in Abständen von 10 mm im mittleren Bereich des Streifens des Trägerbands 11 abgelesen. Diese Messung ergibt sich dann der Durchschnittswert für die Klebkraft.

Aus den exemplarisch in Figur 10B dargestellten Messergebnissen kann im Vergleich zu herkömmlichen Klebstoffen eine deutlich niedrigere Klebkraft für einen Klebstoff, der als Klebstoffschicht 40 nur sehr wenige oder keine Fasern 23 aus den textilen Oberflächenbereichen 22, 26 ausreißt, ermittelt werden.

Insbesondere hat sich herausgestellt, dass die Klebstoffe mit einem Speichermodul *G'* von mehr als 3,3 10⁵ Pa bei 25 °C und einer Glasübergangstemperatur von wenigstens 23 °C wenige bis keine Fasern aus textilen Oberflächenbereichen 22, 26 ausreißen und dennoch eine ausreichende Niederhaltefunktion sowie eine ausreichende starke Befestigung der ersten Komponente 31 des zwei-komponentigen mechanischen Verschlusssystems 30 gewährleisten. Dieses gilt insbesondere, wenn das Speichermodul *G'* mehr als 4,0 10⁵ Pa sowie weniger als 6,0 10⁵ Pa beträgt. Ebenso gilt dieses insbesondere, wenn die Klebstoffschicht eine Glasübergangstemperatur von wenigstens 25 °C, aber weniger als 27,5 °C aufweist.

Wie in Figur 11 exemplarisch dargestellt, unterscheidet sich der ein low-fiberpicking (LFP) ermöglichender Klebstoff 91 von Klebstoffen anderer Art, wie beispielsweise von Klebstoffen 93 für Low-Peel-Label, Klebstoffen 94 für Freezer Label, Klebstoffen 95 für Cold Temp Label, Klebstoffen 96 für PSA tapes (pressure sensitive adhesive tapes), Klebstoffen 97 für High Peel Labels und Klebstoffen 98 für Disposables, da dieser LFP-Klebstoff 91 über dem Dahlquist-Kriterium 90 liegt und eine höhere Glasübergangstemperatur als die übrigen Klebstoffe 93 bis 98 aufweist.

### Bezugszeichenliste:

- 10: Windelverschlussband
- 11: Trägerband
- 12: Permanentklebstoffschicht
- 13: Fingerlift
- 14: Innenseite
- 15: Außenseite
- 16: Abstand zwischen Fastening-Bereich 17 und Permanent-Bereich 18
- 17: Fastening-Bereich
- 18: Permanent-Bereich
- 19: Länge des Windelverschlussbands 10

- 20: Windel
- 21: Trägerschicht
- 22: textiler Oberflächenbereich an der Windelinnenseite 27
- 23: Faser (exemplarisch beziffert)
- 24: Windelrand
- 25: Landezone der Windel 20
- 26: textiler Oberflächenbereich der Landezone 25
- 27: Windelinnenseite
- 28: Windelaußenseite

- 30: zwei-komponentiges mechanisches Verschlusssystem
- 31: erste Komponente des zwei-komponentigen mech. Verschlusssystems 30
- 32: zweite Komponente des zwei-komponentigen mech. Verschlusssystems 30
- 33: Komponentenstreifen
- 34: Breite der Komponentenstreifen 33

- 40: Klebstoffschicht
- 41: offener Bereich der Klebstoffschicht 40
- 42: offener Bereich der Klebstoffschicht 40
- 43: offener Bereich der Klebstoffschicht 40
- 44: Breite der offenen Bereiche 41, 42, 43

- 50: Halbzeugband
- 51: Rolle
- 52: Spule
- 53: Querrichtung
- 54: Maschinenlaufrichtung

- 60: Bewegungsrichtung für peel-Mindestkraftmessung
- 61: obere Klemmbacke eines Zugprüfgeräts
- 62: untere Klemmbacke eines Zugprüfgeräts
- 63: Verstärkungsband

- 70: Bewegungsrichtung für loop-tack-Prüfung
- 71: Breite des für die loop-track-Prüfung genutzten Trägerbands 11
- 72: Prüfplatte für die loop-track-Prüfung
- 73: Breite der Prüfplatte 72
- 74: Länge der Prüfplatte 72

- 80: Bewegungsrichtung für peel-adhesion-Prüfung
- 81: Breite des für die peel-adhesion-Prüfung genutzten Trägerbands 11
- 82: Prüfplatte für peel-adhesion-Prüfung
- 83: Breite der Prüfplatte 82

- 90: Dahlquist-Kriterium
- 91: LFP (low fiber picking)
- 93: Low Peel Label
- 94: Freezer Label
- 95: Cold Temp Label

- 96: PSA tapes
- 97: High Peel Labels
- 98: Disposables

## Patentansprüche

1. Mechanisches Windelverschlussband (10) mit einer klebend wirksamen Niederhaltefunktion, wobei das Windelverschlussband (10) ein sich entlang einer Länge (19) erstreckendes Trägerband (11) mit einem Fastening-Bereich (17) und einem von dem Fastening-Bereich (17) entlang der Länge (19) des Trägerbands (11) versetzt angeordneten Permanent-Bereich (18) aufweist, wobei in dem Fastening-Bereich (17) eine erste Komponente (31) eines zwei-komponentigen mechanischen Verschlusssystems (30) mittels einer Klebstoffschicht (40) an dem Trägerband (11) angebracht ist und diese Klebstoffschicht (40) wenigstens einen offenen Bereich (41,42,43) zu Ausübung der Niederhaltefunktion aufweist, **dadurch gekennzeichnet, dass** die Klebstoffschicht (40)
(i) ein Speichermodul *G'* von mehr als 3,3 10⁵ Pa bei 25 °C und eine Glasübergangstemperatur von wenigstens 23 °C aufweist und/oder
(ii) eine Sofortklebekraft zwischen 1,0 N/(25 mm) und 5,0 N/(25 mm) und eine Klebkraft zwischen 10,0 N/(25 mm) und 25,0 N/(25 mm) aufweist.

2. Windelverschlussband (10) nach Anspruch 1, **dadurch gekennzeichnet, dass** die Klebstoffschicht (40) ein Speichermodul *G'* von mehr als 3,6 10⁵ Pa, insbesondere von mehr als 4,0 10⁵ Pa, aufweist.

3. Windelverschlussband (10) nach Anspruch 1 oder 2, **dadurch gekennzeichnet, dass** die Klebstoffschicht (40) ein Speichermodul *G'* von weniger als 7,0 10⁵ Pa, insbesondere von weniger als 6,0 10⁵ Pa, aufweist.

4. Windelverschlussband (10) nach einem der Ansprüche 1 bis 3, **dadurch gekennzeichnet, dass** die Klebstoffschicht (40) eine Glasübergangstemperatur von wenigstens 24,0 °C, vorzugsweise von wenigstens 25,0 °C, aufweist.

5. Windelverschlussband (10) nach einem der Ansprüche 1 bis 4, **dadurch gekennzeichnet, dass** die Klebstoffschicht (40) eine Glasübergangstemperatur von weniger als 28,0 °C, vorzugsweise von weniger als 27,5 °C, aufweist.

6. Windelverschlussband (10) nach einem der Ansprüche 1 bis 5, **dadurch gekennzeichnet, dass** die Klebstoffschicht (40) eine Sofortklebekraft über 1,5 N/(25 mm), insbesondere über 1,8 N/(25 mm), vorzugsweise gegen Polyethylen, und/oder über 2,0 N/(25 mm), insbesondere über 2,5 N/(25 mm), vorzugsweise gegen Polypropylen, und/oder eine Sofortklebekraft unter 6,0 N/(25 mm), insbesondere unter 5,0 N/(25 mm), vorzugsweise gegen Polypropylen, und/oder unter 4,0 N/(25 mm), insbesondere unter 3,0 N/(25 mm), vorzugsweise gegen Polyethylen, aufweist.

7. Windelverschlussband (10) nach einem der Ansprüche 1 bis 6, **dadurch gekennzeichnet, dass** die Klebstoffschicht (40) eine Klebkraft über 12,0 N/(25 mm), insbesondere über 13,0 N/(25 mm), vorzugsweise gegen Polyethylen, und/oder über 15,0 N/(25 mm), insbesondere über 17,0 N/(25 mm), vorzugsweise gegen Polypropylen, und/oder eine Klebkraft unter 25,0 N/(25 mm), insbesondere unter 23,0 N/(25 mm), vorzugsweise gegen Polypropylen, und/oder unter 20,0 N/(25 mm), insbesondere unter 18,0 N/(25 mm), vorzugsweise gegen Polyethylen, aufweist.

8. Windelverschlussband (10) nach einem der Ansprüche 1 bis 7, **dadurch gekennzeichnet, dass** die Sofortklebekraft durch eine loop-tack-Prüfung, vorzugsweise nach der FINAT-Testmethode FTM 9 und/oder nach einer an die FTM 9 angelehnten Testmethode, zu bestimmen ist und/oder dass die Klebkraft durch eine 180°-peel-adhesion-Prüfung, vorzugsweise nach der FINAT-Testmethode FTM 1 und/oder nach einer an die FTM 1 angelehnten Testmethode, zu bestimmen ist.

9. Halbzeugband (50) zur Bereitstellung eines Windelverschlussbands (10) nach einem der Ansprüche 1 bis 8, wobei das Windelverschlussband (10) durch Trennen des Halbzeugbands (50) parallel zur Länge (19) des Windelverschlussbands (10) aus dem Halbzeugband (50) bereitstellbar ist.

10. Windel (20) mit wenigstens einem öffen- und wieder verschließbaren Windelverschlussband (10) nach einem der Ansprüche 1 bis 8, welches in einem Bereitstellungszustand einen Windelrand (24) der Windel (20) umgreifend sowohl in seinem Permanent-Bereich (18) permanent an der Windel (20) befestigt als auch in seinem Fastening-Bereich (17) lösbar mit der Windel (20) verbunden ist, wobei die Verbindung in dem Fastening-Bereich (17) zu einem textilen Oberflächenbereich (22) der Windel (20) erfolgt und das Windelverschlussband (10) durch Öffnen der Verbindung in dem Fastening-Bereich (17) in einen Arbeitszustand verbringbar ist.

11. Windel (20) mit wenigstens einem öffen- und wieder verschließbaren Windelverschlussband (10), welches ein sich entlang einer Länge (19) erstreckendes Trägerband (11) mit einem Fastening-Bereich (17) und einem von dem Fastening-Bereich (18) entlang der Länge (19) des Trägerbands (11) versetzt angeordneten Permanent-Bereich (18) aufweist, wobei in dem Fastening-Bereich (17) eine erste Komponente (31) eines zwei-komponentigen mechanischen Verschlusssystems (30) mittels einer Klebstoffschicht (40) an dem Trägerband (11) angebracht ist und diese Klebstoffschicht (40) wenigstens einen offenen Bereich (41,42,43) zu Ausübung einer klebend wirksamen Niederhaltefunktion des Windelverschlussbands (10) aufweist, wobei das Windelverschlussband (10) in einem Bereitstellungszustand einen Windelrand (24) der Windel (20) umgreifend sowohl in seinem Permanent-Bereich (18) permanent an der Windel (20) befestigt als auch in seinem Fastening-Bereich (17) lösbar mit der Windel (20) verbunden ist und wobei die Verbindung in dem Fastening-Bereich (17) zu einem textilen Oberflächenbereich (22) der Windel erfolgt und das Windelverschlussband (10) durch Öffnen der Verbindung in dem Fastening-Bereich (17) in einen Arbeitszustand verbringbar ist, **dadurch gekennzeichnet, dass** die Klebstoffschicht (40) einem Öffnen der Verbindung eine peel-Mindestkraft von wenigstens 0,6 N/(25 mm) entgegensetzt und bei dem Öffnen weniger als 20 Fasern (23) pro 0,125 cm² aus dem textilen Oberflächenbereich (22) ausreißt.

12. Windel (20) nach Anspruch 11, **dadurch gekennzeichnet, dass** die Klebstoffschicht (40) einem Öffnen der Verbindung eine peel-Mindestkraft von wenigstens 0,8 N/(25 mm), insbesondere von wenigstens 1,0 N/(25 mm), entgegensetzt und bei dem Öffnen weniger als 20 Fasern (23) pro 0,125 cm² aus dem textilen Oberflächenbereich (22) ausreißt.

13. Windel (20) nach Anspruch 11 oder 12, **dadurch gekennzeichnet, dass** die peel-Mindestkraft wenigstens 0,5 N/(25 mm), vorzugsweise wenigstens 0,7 N/(25 mm), geringer ist als die Maximalkraft, welche das zwei-komponentige mechanische Verschlusssystem (30) dem Öffnen der Verbindung entgegensetzt.

14. Windel (20) nach einem der Ansprüche 11 bis 13, **dadurch gekennzeichnet, dass** zur Bestimmung der peel-Mindestkraft ein 2*90°-Test durchgeführt wird und die Klebstoffschicht (40) vorzugsweise bei diesem Test weniger als 20 Fasern, insbesondere weniger als 17 Fasern (23), insbesondere weniger als 15 Fasern (23) und vorzugsweise keine Fasern (23), aus dem textilen Oberflächenbereich (22) ausreißt.

15. Windel (20) nach einem der Ansprüche 11 bis 14, **dadurch gekennzeichnet, dass** die Klebstoffschicht (40) bei dem Öffnen weniger als 17 Fasern (23), insbesondere weniger als 15 Fasern (23), pro 0,125 cm² aus dem textilen Oberflächenbereich (22) ausreißt.

16. Windel (20) nach einem der Ansprüche 11 bis 15, **dadurch gekennzeichnet, dass** das Windelverschlussband (10) ein Windelverschlussband nach einem der Ansprüche 1 bis 8 ist.

17. Windel (20) nach einem der Ansprüche 10 bis 16, Halbzeugband (50) nach Anspruch 9 oder Windelverschlussband (10) nach einem der Ansprüche 1 bis 8, **dadurch gekennzeichnet, dass** das Windelverschlussband eine Länge (19) kleiner 80 mm, insbesondere kleiner 75 mm, aufweist.

18. Windel (20) nach einem der Ansprüche 10 bis 17, Halbzeugband (50) nach einem der Ansprüche 9, 16 und 17 oder Windelverschlussband (10) nach einem der Ansprüche 1 bis 8, 16 und 17, **dadurch gekennzeichnet, dass** die Klebstoffschicht (40) einen auf der dem Permanent-Bereich (18) zugewandten Seite der ersten Komponente (31) des zwei-komponentigen mechanischen Verschlusssystems (30) angeordneten offenen Bereich (41) aufweist, der vorzugsweise zumindest 0,3 mm, insbesondere zumindest 0,4 mm, und/oder vorzugsweise nicht mehr als 3,0 mm, insbesondere nicht mehr als 2,6 mm, und/oder nicht mehr als 60 %, vorzugsweise nicht mehr als 55 %, des Abstands (16) zwischen dem Fastening-Bereich (17) und dem Permanent-Bereich (18) breit ist.

19. Windel (20) nach einem der Ansprüche 10 bis 18, Halbzeugband (50) nach einem der Ansprüche 9 und 16 bis 18 oder Windelverschlussband (10) nach einem der Ansprüche 1 bis 8 und 16 bis 18, **dadurch gekennzeichnet, dass** die erste Komponente (31) des zwei-komponentigen mechanischen Verschlusssystems (30) wenigstens zwei, vorzugsweise entlang der Länge (19), voneinander beabstandete Komponentenstreifen (33) umfasst und die Klebstoffschicht (40) zwischen den Komponentenstreifen (33) wenigstens einen offenen Bereich (42, 43) aufweist, der vorzugsweise zumindest 0,3 mm, insbesondere zumindest 0,4 mm, und/oder vorzugsweise nicht mehr als 2,5 mm, insbesondere nicht mehr als 2,3 mm, und/oder zumindest 8 %, vorzugsweise zumindest 10 %, und/oder nicht mehr als 100 %, vorzugsweise nicht mehr als 90 %, der Breite (34) eines der Komponentenstreifen (33) breit ist.
